# EUROPEAN PATENT APPLICATION

(11) **EP 3 587 407 A1**
(43) Date of publication of application: **01.01.2020**
(21) Application number: 18758074.1
(22) Date of filing: 27.02.2018
(51) Int. Cl.: C07D 303/04, A23L 2/00, A23L 2/02, A61K 8/49, A61Q 13/00, C11B 9/00

(54) **NOVEL SPIROSESQUITERPENE COMPOUND, FLAVORING COMPOSITION AND FOOD/DRINK CONTAINING SAID COMPOUND, AND METHOD FOR PRODUCING SAID FOOD/DRINK**

(30) Priority: 27.02.2017 JP 2017034869
(71) Applicant: Takasago International Corporation, Tokyo 144-8721 (JP)
(72) Inventor: CHIBA Shingo, Hiratsuka-shi Kanagawa 254-0073 (JP); KURABE Aki, Hiratsuka-shi Kanagawa 254-0073 (JP); KAWARAYA Akihiro, Hiratsuka-shi Kanagawa 254-0073 (JP); KUSANO Yumi, Hiratsuka-shi Kanagawa 254-0073 (JP); YOKOYAMA Naota, Hiratsuka-shi Kanagawa 254-0073 (JP); NAKAZAKI Atsuo, Nagoya-shi Aichi 464-0814 (JP)
(74) Representative: Hoffmann Eitle
(86) International application number: PCT/JP2018/007360
(87) International publication number: WO 2018/155720

(57) **Abstract**

The present invention pertains to a compound represented by formula (1) with which it is possible to impart or enhance an aroma and flavor associated with the feeling of a highly natural fruit or fruit juice, or ripeness. The present invention also pertains to a flavoring composition containing said compound, a food/drink containing said compound or said flavoring composition, and a method for producing said food/drink.

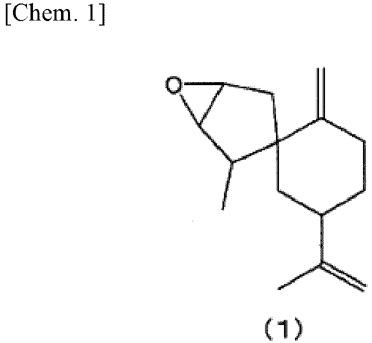

## Description

### TECHNICAL FIELD

The present invention is related to a novel spirosesquiterpene compound, a flavor composition and a food/beverage each containing the compound, and a method for producing the food/beverage. More specifically, the present invention is related to an epoxyspirolepechinene having a woody aroma, a flavor composition and a food/beverage each containing the epoxyspirolepechinene as an effective ingredient, and a method for producing the food/beverage.

### BACKGROUND ART

In recent years, along with a diversification in consumer needs for foods or beverages, a luxury or natural feeling is required of foods or beverages. Color, taste and scent are important factors in judging the deliciousness or natural feeling. Among these, scent plays a key role, and demands for flavors having higher natural feeling are increasing. However, the demands above cannot be sufficiently responded only by a combination of existing flavor materials, and an unprecedented new technology aimed at imparting or enhancing the natural feeling is desired.

As various technologies aimed at imparting or enhancing the natural feeling, for example, Patent Literature 1 discloses a method in which 7,9,11-dodecatrien-4-one, 6,10-undecadien-3-one or 6,8-undecadien-3-one is added to a flavor composition, Patent Literature 2 discloses a method in which 3-mercaptohexanal and 3-mercapto-1-hexanol are added to a flavor composition, and Patent Literature 3 discloses a method in which cis-4,5-epoxy-2E-decenal is added to a flavor composition.

### CITATION LIST

### PATENT LITERATURE

Patent Literature 1: JP-A-2010-83913
Patent Literature 2: JP-A-2008-101097
Patent Literature 3: JP-A-2005-82771

### SUMMARY OF INVENTION

### TECHNICAL PROBLEM

However, in fact, even when a method of adding the above-described compound, etc. to a flavor composition is used, diversified consumer needs cannot be sufficiently responded.

Accordingly, an object of the present invention is to provide a novel compound capable of imparting or enhancing the aroma and flavor giving fruity feeling, fruit juice-like feeling or ripened sensation with high natural feeling, and a flavor composition containing the compound. Another object of the present invention is to provide a food/beverage in which the aroma and flavor giving fruity feeling, fruit juice-like feeling or ripened sensation with high natural feeling are imparted or enhanced by using the compound or composition.

### SOLUTION TO PROBLEM

As a result of intensive studies to attain the objects above, the present inventors have found that a specific spirosesquiterpene compound has the above-described properties, and have accomplished the present invention.

That is, the present invention includes the contents of the following [1] to [12].
[1] A compound represented by the following formula (1): 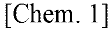 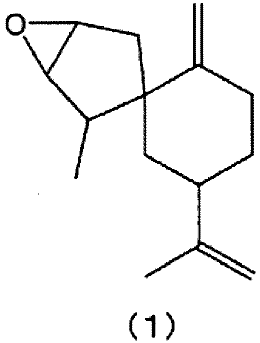
[2] A flavor composition containing the compound according to [1].
[3] The flavor composition according to [2], which is a flavor composition having a fruit-like aroma.
[4] The flavor composition according to [3], wherein the fruit-like aroma is a citrus fruit aroma.
[5] The flavor composition according to [4], wherein the citrus fruit is at least one fruit selected from the group consisting of grapefruit, orange and lemon.
[6] The flavor composition according to any one of [2] to [5], which is a flavor composition for a food/beverage.
[7] The flavor composition according to [6], wherein the food/beverage is a beverage.
[8] The flavor composition according to [7], wherein the beverage is a citrus beverage.
[9] A food/beverage containing the compound according to [1].
[10] A food/beverage containing the flavor composition according to any one of [2] to [8].
[11] A method for producing a food/beverage, including adding the compound according to [1].
[12] A method for producing a food/beverage, including adding the flavor composition according to any one of [2] to [8].

### ADVANTAGEOUS EFFECTS OF INVENTION

According to the present invention, a novel compound capable of imparting or enhancing the aroma and flavor giving fruity feeling, fruit juice-like feeling or ripened sensation with high natural feeling, and a flavor composition containing the compound, can be provided. In addition, according to the present invention, a food/beverage in which the aroma and flavor giving fruity feeling, fruit juice-like feeling or ripened sensation with high natural feeling are imparted or enhanced by using the compound or composition, can be provided.

### DESCRIPTION OF EMBODIMENTS

The embodiments of the present invention are described in detail below.

In the present description, the "compound (X)" means a compound represented by formula (X).

The compound of the present invention is a novel spirosesquiterpene compound represented by the following formula (1):

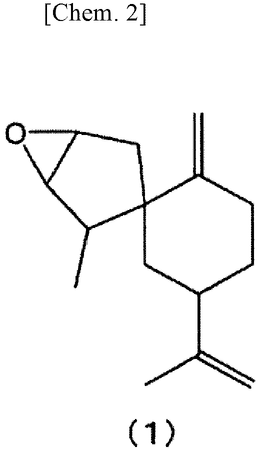

The compound of the present invention encompasses stereoisomers represented by the following formulae (2^{A}) to (2^{P}) and a mixture thereof.

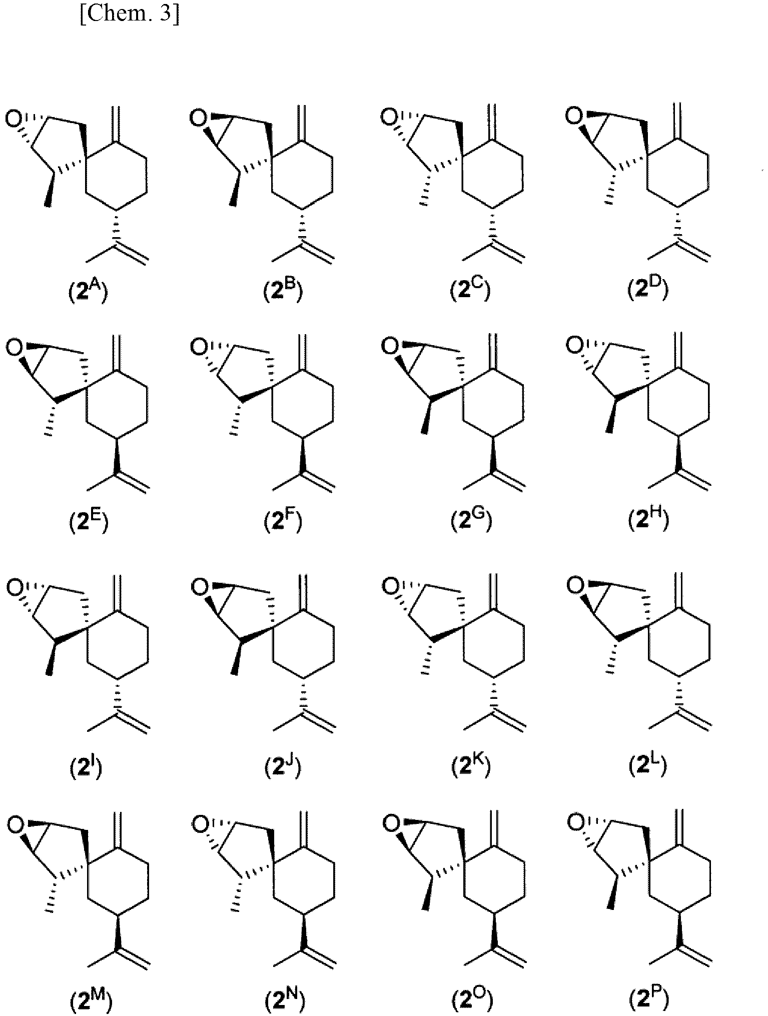

The method for synthesizing the compound of the present invention is described below by referring to a specific example, but the means for synthesizing the compound of the present invention is not limited to this specific example.

As in the synthesis route illustrated below, the compound of the present invention can be synthesized, for example, using perillyl alcohol represented by the following formula (3) as a starting material by appropriately combining various chemical reactions. Various chemical reactions include a carbonylation reaction, a carbon-carbon bond forming reaction, a reduction reaction, an oxidation reaction, an elimination reaction, a silylation reaction, a sulfonylation reaction, a protection/deprotection reaction, a substitution reaction, etc.

The specific method for causing various chemical reactions is described in detail in Examples.

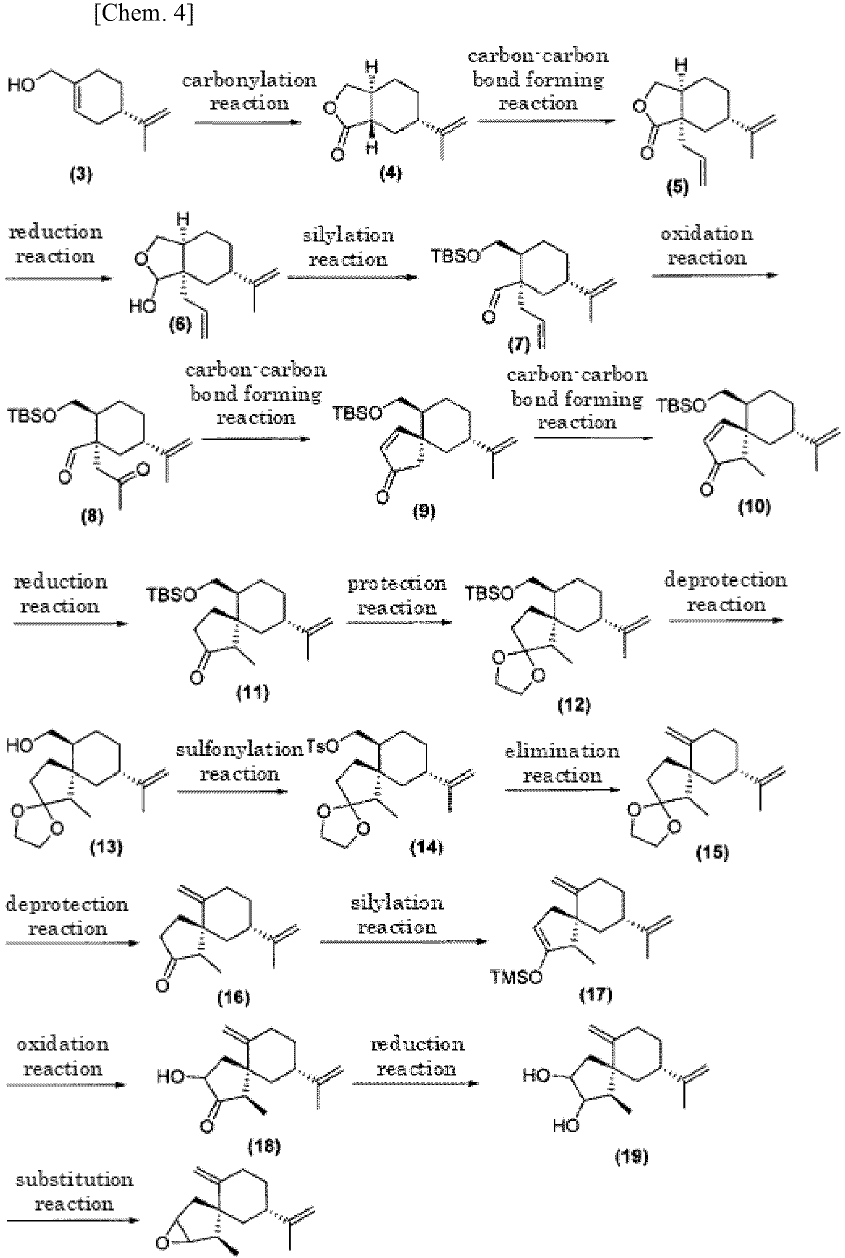

With respect to perillyl alcohol represented by formula (3) serving as a starting material, an R form, an S form, or a mixture thereof at an arbitrary ratio may be used.

The syntheses of intermediates appearing in the synthesis route of the compound of the present invention (hereinafter, referred to as "intermediate") and the synthesis of the compound of the present invention are preferably performed in the presence of a solvent.

Preferable specific examples of the solvent include aliphatic hydrocarbons such as n-pentane, n-hexane, n-heptane, n-octane, n-decane, cyclohexane and decalin, halogenated aliphatic hydrocarbons such as dichloromethane and chloroform, aromatic hydrocarbons such as benzene, toluene, xylene, mesitylene and p-cymene, halogenated aromatic hydrocarbons such as chlorobenzene and o-dichlorobenzene, alcohols such as methanol, ethanol, 2-propanol, n-butanol, tert-butyl alcohol, 2-methyl-2-butanol and 2-ethoxy ethanol, polyols such as ethylene glycol, propylene glycol and glycerin, ethers such as diethyl ether, diisopropyl ether, tert-butyl methyl ether, cyclopentyl methyl ether, 1,2-dimethoxyethane, ethylene glycol diethyl ether, tetrahydrofuran and 1,4-dioxane, amides such as formamide, N,N-dimethylformamide, N,N-dimethylacetamide and N-methylpyrrolidone, nitriles such as acetonitrile and benzonitrile, ketones such as acetone, 3-pentanone and cyclohexanone, sulfoxides such as dimethyl sulfoxide, and water.

More preferable specific examples of the solvent include n-hexane, n-heptane, dichloromethane, toluene, tert-butyl alcohol, 1,2-dimethoxyethane, tert-butyl methyl ether, tetrahydrofuran, N,N-dimethylformamide, N,N-dimethylacetamide, acetonitrile, acetone, and water.

One of these solvents may be used alone, or an appropriate combination of two or more thereof may be used.

The amount used of the solvent is not particularly limited but is appropriately selected in the range of usually from 0.5 to 1,000 times by volume, preferably from 1 to 750 times by volume, more preferably from 3 to 500 times by volume, relative to the intermediate or the compound of the present invention.

The reaction temperature in various chemical reactions for synthesizing intermediates and the compound of the present invention is appropriately selected in the range of usually from -78°C to 200°C, preferably from -78°C to 150°C, more preferably from -78°C to 130°C.

The reaction time in various chemical reactions for synthesizing intermediates and the compound of the present invention varies depending on the type and conditions of the reaction but is appropriately selected in the range of usually from 1 minute to 72 hours, preferably from 2 minutes to 48 hours, more preferably from 5 minutes to 36 hours.

The intermediates and the compound of the present invention, which are obtained in this way, can be subjected to isolation and purification, if desired. The method for isolation and purification includes, for example, column chromatography, distillation, and crystallization, and the isolation and purification may be performed using these methods individually or in combination.

The compound of the present invention has a woody-like aroma, and its use in a trace amount is effective in imparting or enhancing the aroma and flavor giving fruity feel, fruit juice-like feel or ripened sensation with high natural feeling.

The flavor composition of the present invention contains the compound of the present invention.

In addition, the flavor composition of the present invention may contain other flavor ingredients. Other flavor ingredients include various synthetic flavors, natural flavors, natural essential oils, plant extracts, etc., and examples thereof include natural essential oils, natural flavors, and synthetic flavors described in "Patent Office Report: Collection of Well-known Prior Arts (flavors & Fragrances)" (Part II Food Flavors, pp. 88-131, issued January 14, 2000).

The content of the compound represented by formula (1) in the flavor composition of the present invention varies depending on other flavor ingredients contained and cannot be indiscriminately specified, but usually, the compound may be contained at a concentration ranging from 0.0001 to 10,000 ppm, preferably from 0.001 to 1,000 ppm, more preferably from 0.01 to 100 ppm, based on the total mass of the flavor composition.

If the content of the compound represented by formula (1) is less than 0.0001 ppm, the aroma- and flavor-imparting or enhancing effect of the present invention is not obtained, whereas if it exceeds 10,000 ppm, the scent of the flavor composition as a whole may disadvantageously lose its balance.

The flavor composition of the present invention may contain, for example, a solvent such as water and ethanol, and a fixative such as ethylene glycol, propylene glycol, dipropylene glycol, glycerin, hexyl glycol, benzyl benzoate, triethyl citrate, diethyl phthalate, Hercolyn, medium-chain fatty acid triglyceride and medium-chain fatty acid diglyceride, which are usually used as needed.

When the compound of the present invention is added to a flavor composition having a fruit-like aroma and flavor, this is advantageous in that the aroma and flavor giving fruity feel, fruit juice-like feel or ripened sensation with natural feeling can be suitably imparted or enhanced.

The fruit of the "fruit-like" includes, for example, grapefruit, orange, lemon, apple, peach, and grape. Among these, the fruit is preferably at least one citrus fruit selected from the group consisting of grapefruit, orange, and lemon.

The flavor composition of the present invention is preferably a flavor composition for foods and beverages, which is added to a food or beverage.

The flavor composition of the present invention is added to a food or beverage to impart or enhance the aroma and flavor giving fruity feeling, fruit juice-like feeling or ripening feed with natural feeling to the food or beverage.

The amount of the flavor composition of the present invention added to a food or beverage varies according to the type or form of the food or beverage, but usually, the composition may be added at a concentration ranging from 0.001 to 10 mass%, preferably from 0.01 to 5 mass%, based on the mass of the food or beverage before addition of the flavor composition.

If the content of the flavor composition of the present invention is less than 0.001 mass%, the effect of imparting or enhancing the aroma and flavor giving fruity feeling, fruit juice-like feeling or ripened sensation with natural feeling is not obtained, and if the content exceeds 10 mass%, an unpleasant odor is disadvantageously intensified.

In addition, the compound of the present invention can be directly added in a trace amount to a food or beverage to impart or enhance the aroma and flavor giving fruity feeling, fruit juice-like feeling or ripening feed with natural feeling.

In the case of adding the compound to a food or beverage, the compound is added at a concentration ranging from 0.0001 to 10,000 ppb, preferably from 0.001 to 1,000 ppb, more preferably from 0.01 to 100 ppb.

If the content of the compound of the present invention is less than 0.0001 ppb, the effect of imparting or enhancing the aroma and flavor giving fruity feeling, fruit juice-like feeling or ripened sensation with natural feeling is not obtained, and if the content exceeds 10,000 ppb, the balance of aroma and flavor may be disadvantageously lost.

Specific examples of the food and beverage in which the aroma and flavor giving fruity feeling, fruit juice-like feeling or ripened sensation with natural feeling can be imparted or enhanced by the addition of the compound of the present invention and the flavor composition of the present invention include beverages such as carbonated drink, refreshing drink, fruit juice drink, fruit wine, milk beverage, fermented milk drink, health drink, soy milk and tea drink; desserts such as ice cream, ice milk, lacto-ice, frozen confection, yogurt, pudding, jelly and daily dessert; confectionery such as caramel, candy, tablet, cracker, biscuit, cookie, pie, chocolate, snack, chewing gum, steamed bun and sweet bean jelly; soups such as Japanese style soup, Western style soup and Chinese soup; breads; jams; flavorings and seasonings; various instant beverages; and various instant foods.

Among these, beverages are preferred, and a citrus beverage having citrus aroma and flavor is more preferred.

In the case of adding the compound of the present invention to a citrus beverage, the aroma and flavor giving fruity feeling, fruit juice-like feeling or ripened sensation with high natural feeling can be imparted and enhanced in the citrus beverage and therefore, this is particularly preferred.

The citrus of the citrus beverage includes, for example, grapefruit, orange, and lemon.

### EXAMPLES

### (Example 1) Production of Compound of the Present Invention

The production method for the compound of the present invention is specifically described below by referring to Production Examples, but the production method for the compound of the present invention is not limited to these Production Examples.

Unless otherwise indicated, charging of substrate, solvent, etc. was performed under nitrogen stream, and post-treatment of the reaction solution and purification of the crude product were performed in air. In addition, the purity of the compound obtained in the following Production Examples was determined by NMR analysis or gas chromatography analysis.

The apparatuses and conditions used for the measurements of physical properties in Production Examples are as follows.

NMR Measurement apparatus: AVANCE III 500 (manufactured by Bruker Biospin Co. Ltd.)
Gas chromatography measurement apparatus: GC4000 Plus (manufactured by GL Sciences Inc.), column: InertCap 1 (manufactured by GL Sciences Inc.), sample introduction part: 250 °C, sample detection part: 250 °C

[Measurement condition 1] Initial temperature: 50 °C, temperature ramp rate temperature: 10°C/min, final temperature: 250 °C, arrival temperature holding time: 10 minutes
[Measurement condition 2] Initial temperature: 100 °C, temperature ramp rate temperature: 10 °C/min, final temperature: 300 °C, arrival temperature holding time: 10 minutes
[Measurement condition 3] Initial temperature: 100 °C, temperature ramp rate temperature: 10°C/min, final temperature: 300 °C, arrival temperature holding time: 0 minutes

### (Production Example 1)

### Synthesis of (3aS,6S,7aS)-6-(1-propen-2-yl)hexahydroisobenzofuran-1(3H)-one (4) (Eq. 1)

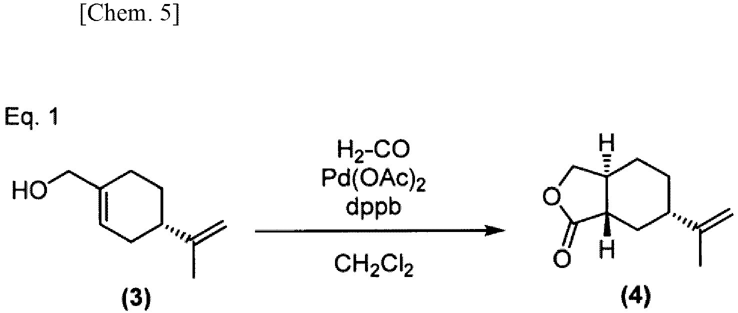

Palladium acetate (0.72 g, 3.2 mmol, 0.08 equivalents), dehydrated dichloromethane (40.0 mL), 1,4-diphenylphosphinobutane (dppb) (1.37 g, 3.2 mmol), and (S)-perillyl alcohol (6.09 g, 40.0 mmol) were charged into a 100 mL four-necked round-bottom flask successively and the solution was stirred at ambient temperature to prepare a substrate solution. After adding 23.0 mL of the substrate solution to a 100 mL autoclave, the inside was pressurized with a mixed gas containing hydrogen and carbon monoxide in equimolar amounts until reaching 5.0 MPa, and the solution was stirred at an external temperature of 110 °C for 9 hours. The autoclave was cooled to ambient temperature, and the gas components were carefully released. The insoluble matter was filtered, and the reaction mixture was then concentrated under reduced pressure. The obtained residue was purified by silica gel column chromatography (eluent: toluene/ethyl acetate=40/1) to obtain 2.15 g (purity: 98.5%, 11.7 mmol) of the title compound as a colorless liquid. The yield was 29.4%.

¹H NMR (500 MHz, pyridine-d₅) δ:
4.75 (2H, m), 4.25 (1H, dd, J=7.9, 6.8 Hz) 3.74 (1H, dd, J=11.0, 8.2 Hz), 2.18 (1H, dt, J=12.6, 3.0 Hz), 2.00 (1H, m), 1.88 (1H, m), 1.79 (1H, m), 1.74-1.67 (2H, m), 1.64 (3H, s), 1.24 (1H, q, J=12.1 Hz), 1.17-1.02 (2H, m)

¹³C NMR (126 MHz, pyridine-ds) δ:
176.75, 148.76, 109.27, 71.64, 44.73, 44.58, 43.00, 30.29, 29.84, 27.18, 20.47

GC retention time (measurement condition 1): 13.0 minutes

### (Production Example 2)

### Synthesis of (3aS,6S,7aR)-7a-allyl-6-(1-propen-2-yl)hexahydroisobenzoiuran-1(3H)-one (5) (Eq. 2)

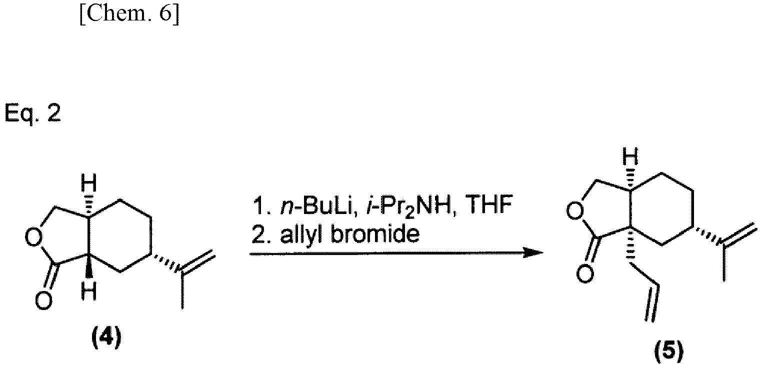

Diisopropylamine (14.3 mL, 102.0 mmol) and dehydrated tetrahydrofuran (THF) (130 mL) were charged into a 1,000 mL four-necked round-bottom flask and the solution was cooled using a dry ice-acetone bath. Subsequently, an n-hexane solution (concentration: 1.55 mol/L, 57.9 mL, 89.7 mmol) of n-butyllithium (n-BuLi) was added dropwise via a dropping funnel at such a speed as to keep an internal temperature of -50 °C or less. The resulting solution was warmed to 0 °C using an ice-water bath and stirred at the same temperature for 30 minutes. The obtained pale yellow solution was cooled using a dry ice-acetone bath. A dehydrated THF (65 mL) solution of the compound (4) (14.70 g, purity: 98.5%, 80.3 mmol) obtained by conducting Production Example 1 a plurality of times was added dropwise via a dropping funnel over 25 minutes at such a speed as to keep an internal temperature of -55 °C or less. After the completion of dropwise addition, dehydrated THF (10 mL) was added to a dropping funnel and added dropwise to the flask. A dehydrated THF (35 mL) solution of allyl bromide (11.84 g, 97.9 mmol) was charged into the same dropping funnel and added dropwise at such a speed as to keep an internal temperature of -60 °C or less. After the completion of dropwise addition, dehydrated THF (10 mL) was added to a dropping funnel and added dropwise to the flask, and the obtained solution was warmed to 0 °C by using an ice-water bath and stirred at the same temperature for 20 minutes. The reaction mixture was cooled using an ice-water bath, and an aqueous 10% ammonium chloride solution (130 mL) was charged thereto, followed by stirring for 20 minutes. The solution was then left standing still, and the aqueous layer was separated. The aqueous layer was extracted twice with diethyl ether (200 mL), and the organic layers were combined and dried over anhydrous sodium sulfate. The insoluble matter was filtered, and the obtained solution was concentrated under reduced pressure to obtain 18.66 g (purity: 90.4%, 76.6 mmol) of a crude product of the title compound as a colorless liquid. The yield was 95.3%.

¹H NMR (500 MHz, pyridine-d₅) δ:
5.85 (1H, m), 5.14 (1H, d, J=1.0 Hz), 5.11 (1H, m), 4.75 (2H, m), 4.37 (1H, dd, J=9.1, 4.8 Hz), 3.78 (1H, d, J=9.1 Hz), 2.36 (1H, dd, J=14.0, 6.7 Hz), 2.29 (1H, dt, J=13.6, 2.7 Hz), 2.23 (1H, dd, 14.0, 8.0 Hz), 2.04 (1H, m), 1.90-1.77 (2H, m), 1.66 (3H, s), 1.55 (1H, m), 1.30 (1H, t, J=13.0 Hz), 1.17 (1H, m), 0.98 (1H, m)

¹³C NMR (126 MHz, pyridine-d₅) δ:
179.20, 149.13, 132.77, 118.81, 108.87, 70.46, 46.73, 41.82, 40.60, 38.88, 34.70, 29.10, 28.62, 20.52

GC retention time (measurement condition 1): 14.5 minutes

### (Production Example 3)

### Synthesis of (3aS,6S,7aR)-7a-allyl-6-(1-propen-2-yl)octahydroisobenzofuran-1-ol (6) (Eq. 3)

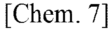

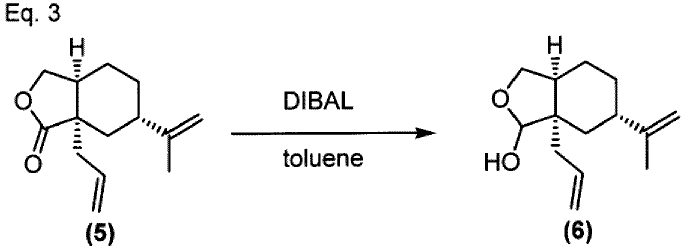

The crude product (18.66 g, purity: 90.4%, 76.6 mmol) of the compound (5) obtained in Production Example 2 and dehydrated toluene (350 mL) were charged into a 1,000 mL four-necked round-bottom flask and cooled using a dry ice-acetone bath. A toluene solution (concentration: 1.00 mol/L, 85.0 mL, 85.0 mmol) of diisobutylaluminum hydride (DIBAL) was charged into a dropping funnel and added dropwise at such a speed as to keep an internal temperature of -55 °C or less, and the solution was then stirred at the same temperature for 40 minutes. After adding methanol (3.0 mL) to the reaction solution, the dry ice-acetone bath was removed. An aqueous 20% potassium sodium tartrate solution (500 mL) was added to the reaction mixture, and the resulting solution was stirred at ambient temperature for 2.5 hours. The solution was then left standing still, and the aqueous layer was separated. The aqueous layer was extracted twice with diethyl ether (100 mL), and the organic layers were combined and dried over anhydrous sodium sulfate. The insoluble matter was filtered, and the obtained solution was concentrated under reduced pressure to obtain 18.60 g (purity: 86.9%, 72.7 mmol) of a crude product of the title compound as a colorless liquid (a mixture of isomers; ratio of isomers: 62.1:37.9). The yield was 95.0%.

GC retention time (measurement condition 1): 14.4 minutes

### (Production Example 4)

### Synthesis of (1R,2S,5S)-1-allyl-2-{[(tert-butyldimethylsilyl)oxy]methyl}-5-(1-propen-2-yl)cyclohexane-1-carboaldehyde (7) (Eq. 4)

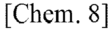

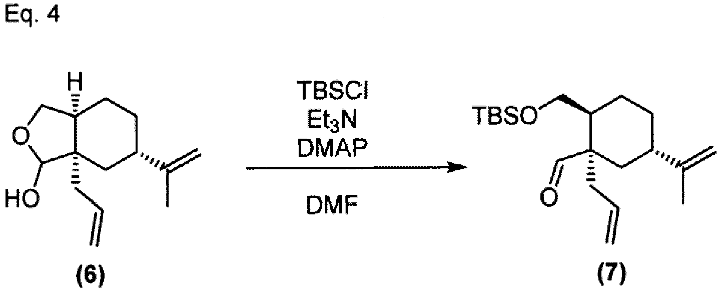

The crude product (18.60 g, purity: 86.9%, 72.7 mmol) of the compound (6) obtained in Production Example 3, dehydrated N,N-dimethylformamide (DMF) (150 mL), triethylamine (Et₃N) (10.30 g, 101.8 mmol), and N,N-dimethyl-4-aminopyridine (DMAP) (0.36 g, 2.9 mmol) were charged into a 1,000 mL four-necked round-bottom flask, and tert-butyl dimethylsilylchloride (TBSCI) (13.15 g, 87.2 mmol) dissolved in dehydrated DMF (100 mL) was added. The mixture was then heated using an oil bath and reacted at an internal temperature of 30 °C for 6 hours. After adding diethyl ether (280 mL) and water (140 mL) to the reaction mixture, the solution was stirred at ambient temperature for 30 minutes. The solution was then left standing still, and the aqueous layer was separated. The aqueous layer was extracted with diethyl ether (140 mL), and the organic layers were combined. After washing with saturated brine (80 mL), the organic layer was dried over anhydrous sodium sulfate, and concentrated under reduced pressure. The obtained residue was purified by silica gel column chromatography (eluent: n-hexane/ethyl acetate=20/1) to obtain 21.70 g (purity: 97.7%, 63.0 mmol) of the title compound as an orange liquid. The yield was 86.6%.

¹H NMR (500 MHz, CDCl₃) δ:
9.89 (1H, s), 5.75 (1H, m), 5.10 (2H, m), 4.69 (2H, m), 3.85 (1H, m), 3.72 (1H, dd, J=10.3, 3.2 Hz), 2.42 (1H, dd, J=14.1, 7.1 Hz), 2.32-2.20 (2H, m), 1.92-1.81 (2H, m), 1.75 (1H, m), 1.71 (3H, s), 1.66 (1H, m), 1.56 (1H, m), 1.29-1.16 (2H, m), 0.88 (9H, s), 0.04 (6H, s)

¹³C NMR (126 MHz, CDCl₃) δ:
206.68, 149.85, 132.95, 118.81, 108.61, 63.26, 51.10, 45.47, 40.72, 39.12, 38.01, 31.31, 26.94, 25.87, 20.85, 18.25, -5.54, -5.58

GC retention time (measurement condition 2): 12.7 minutes

### (Production Example 5)

### Synthesis of (1S,2S,5S)-2-{[(tert-butyldimethylsilyl)oxy]methyl}-1-(2-oxopropyl)-5-(1-propen-2-yl)cyclohexane-1-carboaldehyde (8) (Eq. 5)

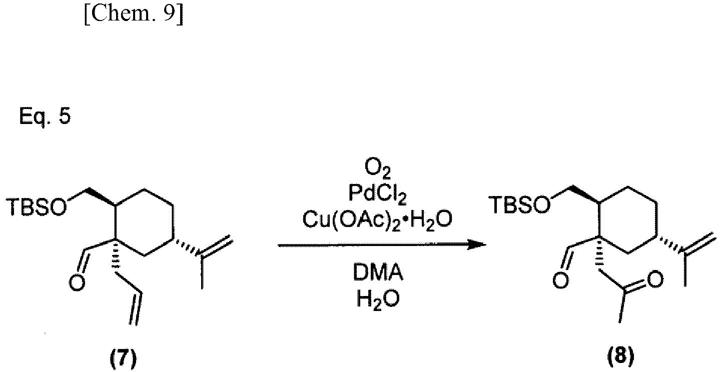

The compound (7) (21.7 g, purity: 97.7%, 63.0 mmol) obtained in Production Example 4, N,N-dimethylacetamide (DMA) (125 mL), distilled water (12.5 mL), palladium chloride (1.12 g, 6.3 mmol) and copper(II) acetate monohydrate (2.52 g, 12.6 mmol) were charged into a 1,000 mL four-necked round-bottom flask, and a balloon filled with 95% oxygen was attached to a three-way stopcock. The inside of the flask was filled with oxygen, and the contents were heated using an oil bath, reacted at an internal temperature of 50 °C for 9 hours. The reaction mixture was then left standing still overnight at room temperature. Palladium chloride (0.56 g, 3.2 mmol) and copper (II) acetate monohydrate (1.26 g, 6.3 mmol) were additionally added and the mixture was reacted at an internal temperature of 50 °C for 4 hours. After cooling the reaction mixture to ambient temperature, diethyl ether (80 mL) and water (120 mL) were added, and the mixture was stirred at ambient temperature for 15 minutes. The insoluble matter was filtered through Celite, and the obtained filtrate was left standing still. After the aqueous layer was separated, the aqueous layer was extracted three times with a mixed solution of hexane (200 mL) and diethyl ether (100 mL) and the organic layers were combined. After washing with saturated brine (100 mL), the organic layer was dried over anhydrous sodium sulfate, and concentrated under reduced pressure. The obtained residue was purified by silica gel column chromatography (eluent: n-hexane/ethyl acetate=40/1) to obtain 11.20 g (purity: 92.3%, 29.3 mmol) of the title compound as a colorless liquid. The yield was 46.5%.

¹H NMR (500 MHz, CDCl₃) δ:
9.96 (1H, s), 4.70 (1H, brs), 4.67 (1H, brs), 3.68 (2H, m), 3.18 (1H, d, J=17.3 Hz), 2.58 (1H, d, J=17.3 Hz), 2.39 (1H, tt, J=12.6, 3.2 Hz), 2.14 (3H, s), 2.10 (1H, m), 1.89 (1H, m), 1.78-1.71 (2H, m), 1.70 (3H, s), 1.56 (1H, m), 1.36-1.23 (2H, m), 0.88 (9H, s), 0.04 (3H, s), 0.03 (3H, s)

¹³C NMR (126 MHz, CDCl₃) δ:
206.93, 205.31, 149.47, 108.86, 64.02, 50.12, 49.92, 46.37, 40.53, 38.49, 31.31, 31.16, 26.79, 25.86, 20.81, 18.25, -5.52, -5.56

GC retention time (measurement condition 2): 14.0 minutes

### (Production Example 6)

### Synthesis of (6S,9S)-6-{[(tert-butyldimethylsilyl)oxy]methyl}-9-(1-propen-2-yl)spiro[4,5]-3-decen-2-one (9) (Eq. 6)

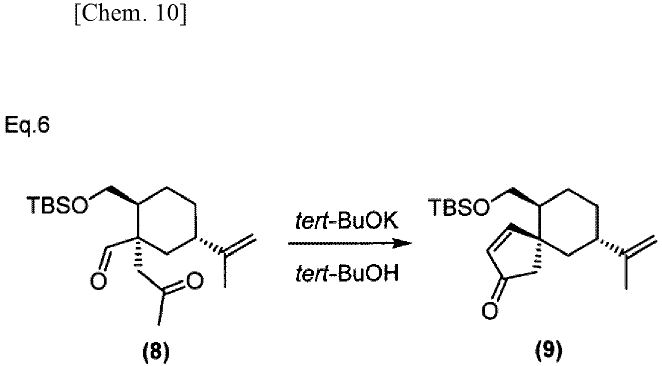

The compound (8) (11.12 g, purity: 92.3%, 29.1 mmol) obtained in Production Example 5, tert-butyl alcohol (tert-BuOH) (220 mL), and potassium tert-butoxide (tert-BuOK) (7.43 g, 66.2 mmol) were charged into a 500 mL four-necked round-bottom flask. The mixture was heated using an oil bath and reacted at an external temperature of 25 °C for 50 minutes. The reaction mixture was cooled to 5 °C or less using an ice-water bath, and toluene (200 mL) and water (100 mL) were added thereto. After the resulting solution was stirred for 10 minutes, the solution was then left standing still, and the aqueous layer was separated. The aqueous layer was extracted twice with toluene (100 mL), and the organic layers were combined. After washing with an aqueous 10% ammonia chloride solution (50 mL), the organic layer was dried over anhydrous sodium sulfate, and concentrated under reduced pressure. The obtained residue was purified by silica gel column chromatography (eluent: n-hexane/ethyl acetate=15/1) to obtain 7.67 g (purity: 98.5%, 22.6 mmol) of the title compound as a colorless liquid. The yield was 72.6%.

¹H NMR (500 MHz, CDCl₃) δ:
7.84 (1H, d, J=5.8 Hz), 6.12 (1H, d, J=5.8 Hz), 4.70 (2H, d, J=13.5 Hz), 3.42 (1H, dd, J=10.4, 5.2 Hz), 3.29 (1H, dd, J=10.4, 5.9 Hz), 2.74 (1H, d, J=18.8 Hz), 2.17 (1H, tt, J=11.8, 3.8 Hz), 2.04 (1H, d, J=18.8 Hz), 1.96-1.85 (2H, m), 1.75-1.67 (4H, m), 1.63 (2H, m), 1.43 (1H, qd, J=12.9, 3.2 Hz), 1.32 (1H, qd, J=12.9, 3.2 Hz), 0.85 (9H, s), -0.02 (6H, s)

¹³C NMR (126 MHz, CDCl₃) δ:
208.69, 167.73, 149.19, 133.43, 109.00, 64.70, 49.00, 48.19, 46.02, 45.32, 41.39, 31.16, 26.65, 25.85, 20.79, 18.18, -5.57, -5.60

GC retention time (measurement condition 2): 14.4 minutes

### (Production Example 7)

### Synthesis of (1R,6S,9S)-6-{[(tert-butyldimethylsilyl)oxy]methyl}-1-methyl-9-(1-propen-2-yl)spiro[4,5]-3-decen-2-one (10) (Eq. 7)

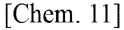

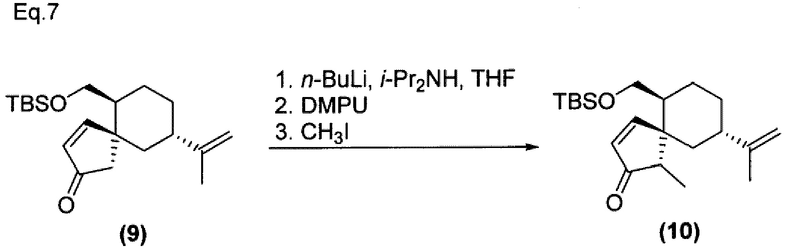

Diisopropylamine (3.3 mL, 23.8 mmol) and dehydrated THF (74 mL) were added to a 300 mL four-necked round-bottom flask and cooled using an ice-water bath. While keeping the internal temperature at 5 °C or less, an n-hexane solution (concentration: 1.55 mol/L, 13.8 mL, 21.5 mmol) of n-BuLi was added dropwise. After stirring at the same temperature for 30 minutes, a dehydrated THF (36 mL) solution of the compound (9) (3.60 g, purity: 98.5%, 10.6 mmol) obtained in Production Example 6 was added dropwise, followed by stirring for 30 minutes. The reaction mixture was then cooled using a dry ice-acetone bath and while keeping the internal temperature at -50°C or less, N,N'-dimethylpropyleneurea (DMPU) (13.0 mL, 108.0 mmol) was added. Subsequently, a tert-butyl methyl ether solution (concentration: 2.03 mol/L, 8.0 mL, 16.2 mmol) of methyl iodide (CH₃I) was added dropwise. After the completion of dropwise addition, the solution was stirred at an internal temperature of -10 °C for 4 hours. The reaction mixture was cooled using an ice-water bath, and an aqueous 5% ammonium chloride solution (50 mL) and diethyl ether (100 mL) were added. After the resulting solution was stirred for 10 minutes, the solution was then left standing still, and the aqueous layer was separated. The organic layer was washed twice with an aqueous 5% ammonium chloride solution (25 mL) and then dried over anhydrous sodium sulfate. After concentration under reduced pressure, the obtained residue was purified by silica gel column chromatography (eluent: n-hexane/ethyl acetate=25/1) to obtain 1.92 g (purity: 98.5%, 5.43 mmol) of the title compound as a colorless liquid. The yield was 51.2% (a mixture of isomers; ratio of isomers: 85.5:14.5).

¹H NMR (main product) (500 MHz, CDCl₃) δ:
7.92 (1H, d, J=6.0 Hz), 6.12 (1H, d, J=6.0 Hz), 4.70 (1H, brs), 4.67 (1H, brs), 3.48 (1H, dd, J=10.5, 4.3 Hz), 3.41 (1H, 10.5, 4.6 Hz), 2.79 (1H, q, J=7.3 Hz), 2.11 (1H, tt, J=12.4, 3.0 Hz), 1.93 (1H, m), 1.89 (1H, m), 1.69 (3H, s), 1.68-1.53 (3H, m), 1.36-1.22 (2H, m), 1.06 (3H, d, J=7.4 Hz), 0.85 (9H, s), -0.02 (3H, s), -0.03 (3H, s)

¹³C NMR (main product) (126 MHz, CDCl₃) δ:
211.27, 168.05, 149.55, 131.30, 108.84, 64.81, 50.94, 48.77, 43.97, 42.20, 40.86, 31.32, 26.76, 25.81, 20.89, 18.12, 9.31, -5.59, -5.62

GC retention time (measurement condition 2): 14.5 minutes (minor product), 14.6 minutes (main product)

The main product was a compound represented by the following formula (10^{A}):

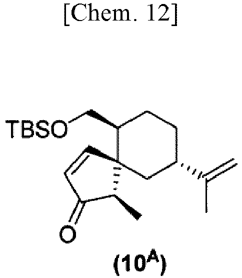

### (Production Example 8)

### Synthesis of (1R,6S,9S)-6-{[(tert-butyldimethylsilyl)oxy]methyl}-1-methyl-9-(1-propen-2-yl)spiro[4,5]decan-2-one (11) (Eq. 8)

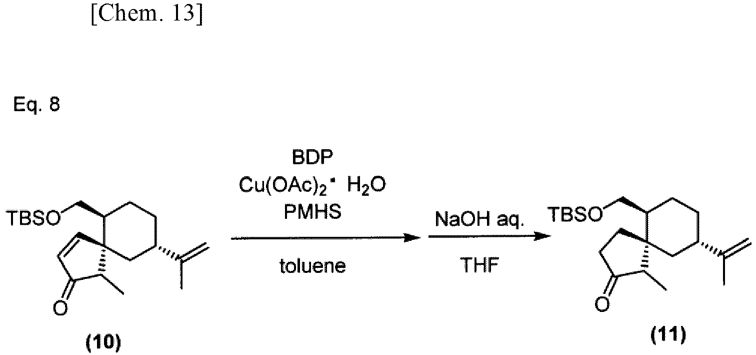

1,2-Bis(diphenylphosphino)benzene (BDP) (80.0 mg, 0.18 mmol), copper(II) acetate monohydrate (200.0 mg, 1.0 mmol), dehydrated toluene (30 mL), and polymethylhydrosiloxane (PMHS) (4.5 mL) were added to a 200 mL four-necked round-bottom flask. After the mixture was stirred at ambient temperature for 1 hour, a dehydrated toluene (15 mL) solution of the compound (10) (3.08 g, purity: 98.5%, 8.70 mmol) obtained by conducting Production Example 7 a plurality of times was added dropwise via a dropping funnel. After the completion of dropwise addition, the solution was stirred at ambient temperature for 1 hour. The reaction mixture was filtered through Celite. After the residue was washed with toluene (20 mL), the filtrate was concentrated under reduced pressure. The obtained crude product was transferred to a 500 mL eggplant flask and dehydrated THF (11 mL) was added to the flask. After the mixture was cooled using an ice-water bath, an aqueous 3.0 mol/L sodium hydroxide solution (NaOH aq.) (200 mL) was added dropwise. After the completion of dropwise addition, the solution was stirred at ambient temperature for 30 minutes, and water (100 mL) and diethyl ether (50 mL) were added. The resulting solution was stirred for 15 minutes and left standing still, and the aqueous layer was separated. The organic layer was washed sequentially with an aqueous 5% ammonium chloride solution (30 mL) and with water (30 mL). The organic layer was dried over anhydrous sodium sulfate and then concentrated under reduced pressure, and the residue was purified by silica gel column chromatography (eluent: n-hexane/ethyl acetate=15/1) to obtain 2.86 g (purity: 99.6%, 8.12 mmol) of the title compound as a colorless liquid. The yield was 93.4% (a mixture of isomers; ratio of isomers: 80.6:19.4).

¹H NMR (main product) (500 MHz, CDCl₃) δ:
4.69 (1H, brs), 4.66 (1H, brs), 3.77 (1H, dd, J=10.5, 6.1 Hz), 3.49 (1H, dd, J=10.5, 5.7 Hz), 2.67 (1H, q, J=6.8 Hz), 2.28 (1H, dd, J=19.2, 9.7 Hz), 2.18-2.07 (2H, m), 2.00 (1H, m), 1.84 (1H, m), 1.81-1.74 (2H, m), 1.69 (3H, s), 1.59 (1H, m), 1.35-1.14 (2H, m), 1.14-1.07 (2H, m), 0.95 (3H, d, J=6.9 Hz), 0.88 (9H, s), 0.04 (3H, s), 0.03 (3H, s)

¹³C NMR (main product) (126 MHz, CDCl₃) δ:
220.58, 149.79, 108.68, 64.32, 51.98, 45.27, 43.34, 40.09, 34.69, 34.00, 31.43, 26.02, 25.87, 23.41, 21.00, 18.19, 6.92, -5.46, -5.52

GC retention time (measurement condition 2): 14.6 minutes (minor product), 14.7 minutes (main product)

The main product was a compound represented by the following formula (11^{A}):

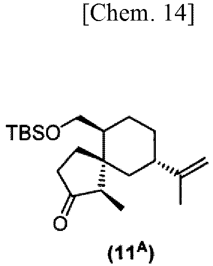

### (Production Example 9)

### Synthesis of tert-butyldimethyl{[(6R,7S,8S,11S)-6-methyl-11-(1-propen-2-yl)-1,4-dioxadispiro[4,1,5⁷2⁵]tetradecan-8-yl]methoxylsilane (12) (Eq. 9)

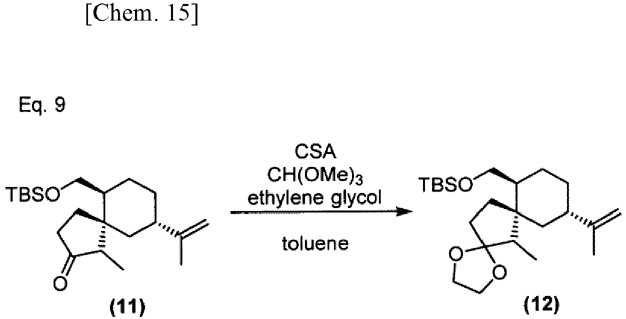

The compound (11) (1.26 g, purity: 99.6%, 3.58 mmol) obtained in Production Example 8, dehydrated toluene (110 mL), trimethyl orthoformate (2.28 g, 21.5 mmol), and ethylene glycol (1.33 g, 21.5 mmol) were added to a 200 mL four-necked round-bottom flask, and L-camphorsulfonic acid (CSA) (0.17 g, 0.72 mmol) was then added. The mixture was stirred at ambient temperature for 8 hours and left standing still overnight. Subsequently, to an aqueous 10% potassium carbonate solution (40 g) cooled to 5 °C or less using an ice-water bath, the reaction mixture was added. The resulting solution was stirred at the same temperature for 10 minutes and then left standing still, and the aqueous layer was separated. The aqueous layer was extracted three times with toluene (30 mL), and the organic layers were combined. The organic layer was dried over anhydrous sodium sulfate and then concentrated under reduced pressure. The obtained residue was purified by silica gel column chromatography (eluent: n-hexane/ethyl acetate/triethylamine=50/1/0.5) to obtain 0.94 g (purity: 98.6%, 2.35 mmol) of the title compound as a colorless liquid. The yield was 65.6% (a mixture of isomers; ratio of isomers: 95.7:4.3).

¹H NMR (main product) (500 MHz, CDCl₃) δ:
4.67 (2H, m), 3.93-3.86 (3H, m), 3.79 (2H, m), 3.36 (1H, dd, J=10.1, 7.5 Hz), 2.31 (1H, q, 7.2), 2.01 (1H, m), 1.91 (1H, m), 1.81-1.72 (2H, m), 1.71 (3H, s), 1.71-1.49 (4H, m), 1.39 (1H, m), 1.23-1.09 (2H, m), 1.04 (1H, t, J=12.8), 0.88 (9H, s), 0.81 (3H, d, J=7.0 Hz), 0.03 (6H, s)

¹³C NMR (main product) (126 MHz, CDCl₃) δ:
150.92, 117.87, 108.12, 65.19, 64.21, 64.19, 45.67, 44.63, 43.98, 40.40, 37.44, 35.16, 31.50, 27.45, 26.96, 25.94, 21.04, 18.26, 6.62, -5.38, -5.39

GC retention time (measurement condition 2): 15.6 minutes (main product), 15.8 minutes (minor product)

The main product was a compound represented by the following formula (12^{A}):

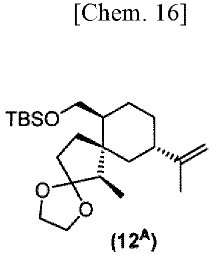

### (Production Example 10)

### Synthesis of [(6R,7S,8S,11S)-6-methyl-11-(1-propen-2-yl)-1,4-dioxadispiro[4,1,5⁷2⁵]tetradecan-8-yl]methanol (13) (Eq. 10)

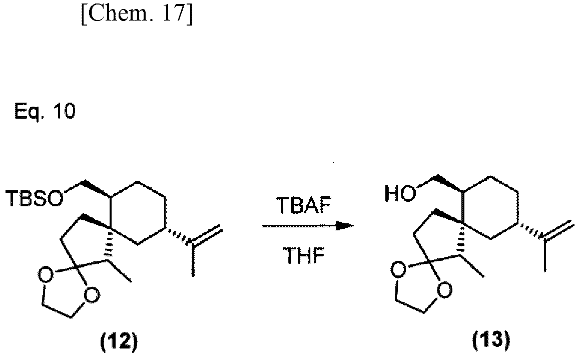

The compound (12) (1.12 g, purity: 98.6%, 2.84 mmol) obtained by conducting Production Example 9 a plurality of times, dehydrated THF (28 mL), and a THF solution (concentration: 1.00 mol/L, 5.7 mL, 5.7 mmol) of tetrabutylammnonium fluoride (TBAF) were added to a 100 mL four-necked round-bottom flask. The mixture was stirred at ambient temperature for 3 hours and then left standing still overnight. Diethyl ether (30 mL) and water (15 mL) were added to the reaction mixture. After stirring for 10 minutes, the solution was then left standing still, and the aqueous layer was separated. The aqueous layer was extracted with diethyl ether (30 mL), and the organic layers were combined. After washing with saturated brine (20 mL), the organic layer was then dried over anhydrous sodium sulfate, and concentrated under reduced pressure. The obtained residue was purified by silica gel column chromatography (eluent: n-hexane/ethyl acetate/triethylamine=4/1/0.05) to obtain 0.73 g (purity: 95.2%, 2.48 mmol) of the title compound as a colorless liquid. The yield was 88.6% (a mixture of isomers; ratio of isomers: 95.0:5.0).

¹H NMR (main product) (500 MHz, CDCl₃) δ:
4.68 (2H, m), 3.96-3.86 (4H, m), 3.80 (1H, m), 3.38 (1H, t, J=9.2 Hz), 2.28 (1H, q, J=7.1 Hz), 2.02 (1H, m), 1.96 (1H, m), 1.81-1.75 (2H, m), 1.72 (3H, s), 1.68 (1H, m), 1.66 (1H, m), 1.54 (2H, m), 1.42 (1H, m), 1.23-1.12 (2H, m), 1.06 (1H, t, J=12.8), 0.83 (3H, d, J=7.1 Hz)

¹³C NMR (main product) (126 MHz, CDCl₃) δ:
150.66, 117.74, 108.26, 65.21, 64.20, 64.18, 45.84, 44.62, 44.34, 40.30, 37.27, 35.13, 31.32, 27.49, 27.05, 21.04, 6.72

GC retention time (measurement condition 2): 13.6 minutes (main product), 13.8 minutes (minor product)

The main product was a compound represented by the following formula (13^{A}):

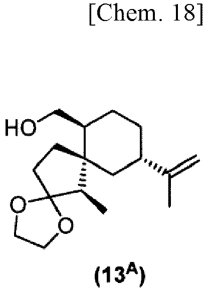

### (Production Example 11)

### Synthesis of [(6R,7S,8S,11S)-6-methyl-11-(1-propen-2-yl)-1,4-dioxadispiro[4,1,5⁷,2⁵]tetradecan-8-yl]methyl-4-methylbenzenesulfonate (14) (Eq. 11)

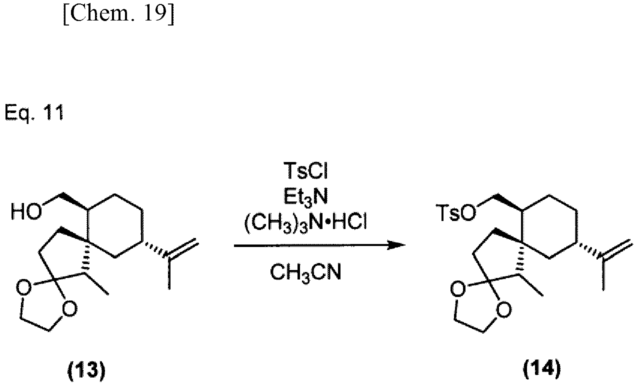

The compound (13) (0.73 g, purity: 95.2%, 2.48 mmol) obtained in Production Example 10, dehydrated acetonitrile (CH₃CN) (5.2 mL), Et₃N (0.66 g, 6.5 mmol), and trimethylamine hydrochloride (24.9 mg, 0.26 mmol) were added to a 100 mL four-necked round-bottom flask, and the mixture was cooled to an internal temperature of 5 °C or less by using an ice-water bath. Subsequently, p-toluenesufonyl chlorode (TsCl) (0.75 g, 3.9 mmol) was added, and the mixture was stirred at the same temperature for 15 minutes. Ethyl acetate (25 mL) and water (7 mL) were added to the reaction mixture. After the resulting solution was stirred for 10 minutes, the solution was then left standing still, and the aqueous layer was separated. The aqueous layer was extracted with ethyl acetate (10 mL), and the organic layers were combined. After washing with saturated brine (10 mL), the organic layer was then dried over anhydrous sodium sulfate, and concentrated under reduced pressure. The obtained residue was purified by silica gel column chromatography (eluent: n-hexane/ethyl acetate/triethylamine=7/1/0.08) to obtain 1.07 g (purity: 99% or more, 2.46 mmol) of the title compound as a colorless liquid. The yield was 99.3% (a mixture of isomers; ratio of isomers: 95.0:5.0).

¹H NMR (main product) (500 MHz, CDCl₃) δ:
7.78 (2H, m), 7.34 (2H, m), 4.66 (2H, m), 4.20 (1H, dd, J=9.7, 3.9 Hz), 3.91-3.82 (3H, m), 3.80-3.74 (2H, m), 2.45 (3H, s), 2.03-1.93 (2H, m), 1.87 (1H, m), 1.79-1.53 (6H, m), 1.69 (3H, s), 1.35 (1H, m), 1.19-1.04 (2H, m), 1.00 (1H, t, J=13.0 Hz), 0.74 (3H, d, J=7.1 Hz)

¹³C NMR (main product) (126 MHz, CDCl₃) δ:
150.15, 144.68, 133.11, 129.86, 127.85, 117.26, 108.51, 72.04, 65.24, 64.18, 46.18, 44.58, 41.07, 40.00, 36.63, 35.00, 30.85, 27.25, 26.79, 21.64, 20.99, 6.43

The compound (14) obtained in this Production Example was decomposed under the gas chromatography measurement conditions and therefore, was not measured for the GC retention time.

The main product was a compound represented by the following formula (14^{A}):

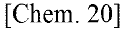

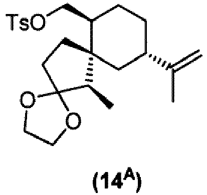

### (Production Example 12)

### Synthesis of (6R,7S, 11S)-6-methyl-8-methylene-11-(1-propen-2-yl)-1,4-dioxadispiro[4,1,5⁷,2⁵]tetradecane (15) (Eq. 12)

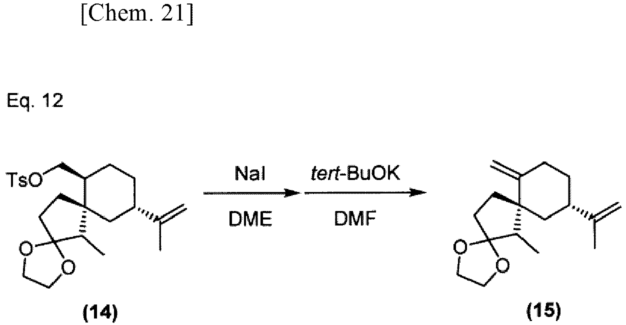

The compound (14) (1.07 g, purity: 99% or more, 2.46 mmol) obtained in Production Example 11, dehydrated ethylene glycol dimethyl ether (DME) (50 mL), and sodium iodide (NaI) (1.11 g, 7.42 mmol) were added to a 100 mL four-necked round-bottom flask, and the mixture was stirred at an external temperature of 90 °C for 2 hours by using an oil bath. The oil bath was removed, and the mixture was cooled to ambient temperature. To the flask, dehydrated DMF (25 mL) and tert-BuOK (0.83 g, 7.42 mmol) were added. After the mixture was stirred at an external temperature of 100 °C for 30 minutes by using an oil bath, the reaction mixture was cooled to ambient temperature. Diethyl ether (25 mL) and water (25 mL) were added thereto. After stirring for 10 minutes, the solution was then left standing still, and the aqueous layer was separated. The aqueous layer was extracted with diethyl ether (60 mL), and the organic layers were combined. The organic layer was dried over anhydrous sodium sulfate, and concentrated under reduced pressure. The obtained residue was purified by silica gel column chromatography (eluent: n-hexane/ethyl acetate/triethylamine=50/1/0.5) to obtain 0.58 g (purity: 98.9%, 2.19 mmol) of the title compound as a colorless liquid. The yield was 88.8% (a mixture of isomers; ratio of isomers: 94.7:5.3).

¹H NMR (main product) (500 MHz, CDCl₃) δ:
4.69 (2H, m), 4.66 (1H, brs), 4.59 (1H, brs), 3.97-3.85 (3H, m), 3.80 (1H, m), 2.44 (1H, q, J=7.1 Hz), 2.30-2.23 (3H, m), 2.08 (1H, m), 1.82 (2H, m), 1.80 (1H, m), 1.73 (1H, m) 1.72 (3H, brs), 1.42 (1H, m), 1.23 (1H, m), 1.14 (1H, t, J=12.7 Hz), 0.88 (3H, d, J=7.1 Hz)

¹³C NMR (main product) (126 MHz, CDCl₃) δ:
153.67, 150.08, 118.22, 108.61, 104.24, 65.22, 64.07, 48.48, 46.55, 40.72, 35.39, 34.97, 34.16, 33.54, 33.19, 20.96, 7.08

GC retention time (measurement condition 2): 11.1 minutes (main product), 11.6 minutes (minor product)

The main product was a compound represented by the following formula (15^{A}):

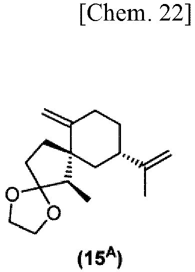

### (Production Example 13)

### Synthesis of (1R,5R,9S)-1-methyl-6-methylene-9-(1-propen-2-yl)-spiro[4,5]decan-2-one (16) (Eq. 13)

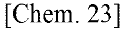

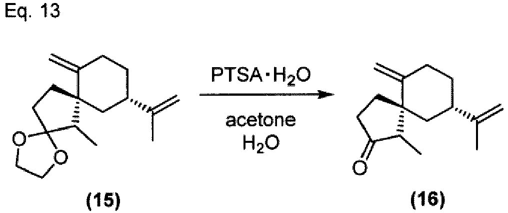

The compound (15) (0.58 g, purity: 98.9%, 2.19 mmol) obtained in Production Example 12, acetone (45 mL), water (22 mL), and p-toluenesulfonic acid monohydrate (PTSA·H₂O) (84.5 mg, 0.44 mmol) were added to a 100 mL four-necked round-bottom flask, and the mixture was stirred at an external temperature of 20 °C for 9 hours by using an oil bath. Diethyl ether (150 mL) and an aqueous 5% sodium hydrogencarbonate solution (40 g) were added to the reaction mixture. After the resulting solution was stirred for 10 minutes, the solution was then left standing still, the aqueous layer was separated. The aqueous layer was extracted twice with diethyl ether (50 mL), and the organic layers were combined. The organic layer was dried over anhydrous sodium sulfate and concentrated under reduced pressure. The obtained residue was purified by silica gel column chromatography (eluent: n-hexane/ethyl acetate=40/1) to obtain 0.44 g (purity: 99.2%, 2.00 mmol) of the title compound as a colorless liquid. The yield was 91.4% (a mixture of isomers; ratio of isomers: 94.6:5.4).

¹H NMR (main product) (500 MHz, CDCl₃) δ:
4.77 (1H, brs), 4.71 (1H, m), 4.69 (1H, m), 4.60 (1H, brs), 2.56 (1H, q, J=7.0 Hz), 2.42 (1H, ddd, J=12.9, 9.1, 2.5 Hz), 2.38-2.29 (4H, m), 2.17 (1H, m), 1.89 (1H, m), 1.70 (3H, s), 1.60 (1H, m), 1.33-1.17 (3H, m), 1.02 (3H, d, J=7.1 Hz)

¹³C NMR (main product) (126 MHz, CDCl₃) δ:
220.37, 152.30, 149.19, 109.10, 105.53, 51.60, 48.36, 40.57, 34.33, 34.15, 33.21, 32.88, 30.53, 20.93, 8.25

GC retention time (measurement condition 2): 9.3 minutes (main product), 9.5 minutes (minor product)

The main product was a compound represented by the following formula (16^{A}):

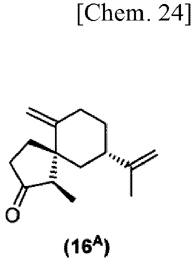

### (Production Example 14)

### Synthesis of (1R,9S)-3-hydroxy-1-methyl-6-methylene-9-(1-propen-2-yl)-spiro[4,5]decan-2-one (18) (Eq. 14)

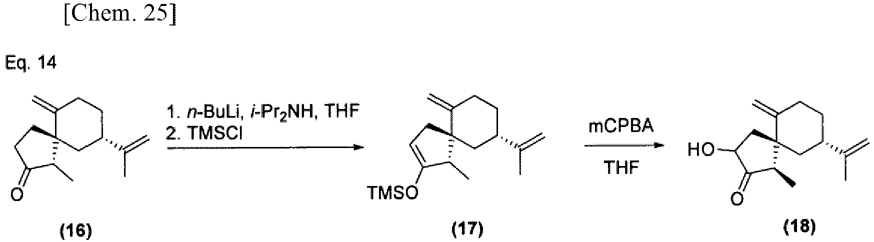

Diisopropylamine (0.56 g, 5.5 mmol) and dehydrated tetrahydrofuran (THF) (17 mL) were added to a 100 mL four-necked round-bottom flask, and the mixture was cooled using an ice-water bath. A hexane solution (concentration: 1.55 mol/L, 3.6 mL, 5.5 mmol) of n-BuLi was added dropwise via a dropping funnel at such a speed as to keep an internal temperature of 5 °C or less. The resulting solution was stirred at the same temperature for 30 minutes and then cooled using a dry ice-acetone bath. While keeping the internal temperature at -50 °C or less, a dehydrated THF (8 mL) solution of the compound (16) (0.36 g, purity: 99.2%, 1.64 mmol) obtained in Production Example 13 was added dropwise. After stirring for 30 minutes, a dehydrated THF (3 mL) solution of chlorotrimethylsilane (TMSC1) (0.75 g, 5.0 mmol) was added dropwise over 5 minutes at the same temperature. The resulting solution was stirred for 1 hour at the same temperature and then warmed to 0 °C by using an ice-water bath. Diethyl ether (50 mL) and an aqueous 5% sodium hydrogencarbonate solution (15 g) were added to the reaction mixture. After stirring for 10 minutes, the solution was then left standing still, and the aqueous layer was separated. The aqueous layer was extracted with diethyl ether (50 mL), and the organic layers were combined. The organic layer was washed with saturated brine (10 mL), and dried over anhydrous sodium sulfate. After filtering the insoluble matter, the reaction mixture was concentrated under reduced pressure to obtain a crude product of the compound (17). Dehydrated THF (17 mL) was added to the obtained crude product of the compound (17), and the resulting solution was cooled to 5 °C inside by using an ice-water bath. Thereafter, meta-chloroperbenzoic acid (mCPBA) (purity: 71.8%, 0.60 g, 2.49 mmol) was added, and the ice-water bath was removed. After stirring for 1 hour at ambient temperature, the reaction mixture was cooled to 5 °C or less using an ice-water bath. After an aqueous 10% sodium thiosulfate solution (10 mL) and 0.1 mol/L hydrochloric acid (30 mL) were added, the ice-water bath was removed, and the solution was stirred for 30 minutes at ambient temperature. Ethyl acetate (60 mL) was added to the resulting solution and the solution was stirred for 5 minutes. The solution was then left standing still, and the aqueous layer was separated. The aqueous layer was extracted with ethyl acetate (60 mL), and the organic layers were combined and washed with an aqueous 10% sodium carbonate solution (20 mL) and saturated brine (20 mL). The organic layer was dried over anhydrous sodium sulfate and concentrated under reduced pressure. The obtained residue was purified by silica gel column chromatography (eluent: n-hexane/ethyl acetate=8/1) to obtain 0.14 g (purity: 90.9%, 0.54 mmol) of the title compound as a colorless liquid. The yield was 33.2% (a mixture of isomers; ratio of isomers at 3-position: 96.0:4.0).

¹H NMR (main product) (500 MHz, CDCl₃) δ:
4.78 (1H, brs), 4.70 (1H, m), 4.68 (1H, m), 4.60 (1H, brs), 4.21 (1H, ddd, J=9.4, 4.3, 1.7 Hz), 2.88 (1H, qd, J=7.0, 1.5 Hz), 2.37 (1H, m), 2.33 (2H, m), 2.24 (1H, dd, J=14.1, 4.3 Hz), 2.13 (1H, ddd, J=14.1, 9.4, 1.4 Hz), 1.88 (1H, m), 1.70 (3H, s), 1.44 (1H, dt, J=13.1, 2.7), 1.30-1.20 (2H, m), 1.07 (3H, d, J=7.0 Hz)

¹³C NMR (main product) (126 MHz, CDCl₃) δ:
219.39, 152.04, 149.14, 109.01, 105.83, 71.19, 48.88, 46.36, 40.11, 38.98, 37.14, 33.18, 32.62, 21.00, 8.30

GC retention time (measurement condition 3): 10.5 minutes (main product), 10.7 minutes (minor product)

The main product was a compound represented by the following formula (18^{A}):

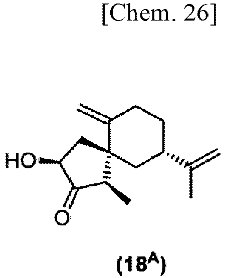

### (Production Example 15)

### Synthesis of (1R,9S)-1-methyl-6-methylene-9-(1-propen-2-yl)-spiro[4,5]decane-2,3-diol (19) (Eq. 15)

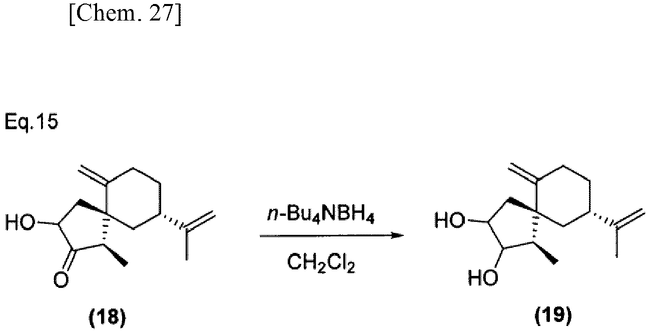

A dehydrated dichloromethane (6.0 mL) solution of the compound (18) (0.14 g, purity: 90.9%, 0.54 mmol) obtained in Production Example 14 and tetrabutylammonium borohydride (purity: 97.5%, 0.46 g, 1.81 mmol) were added to a 50 mL three-necked round-bottom flask, and the mixture was stirred at ambient temperature for 2 hours and 30 minutes. A 3% hydrogen peroxide solution (20 mL) and an aqueous 10% sodium hydroxide solution (10 mL) were added to the reaction mixture. After the resulting solution was stirred for 5 minutes, the solution was then left standing still, and the aqueous layer was separated. The aqueous layer was extracted twice with dichloromethane (35 mL), and the organic layers were combined and washed with an aqueous 10% sodium thiosulfate solution (5 mL) and an aqueous saturated sodium sulfate solution (5 mL). The organic layer was dried over anhydrous sodium sulfate and concentrated under reduced pressure. The obtained residue was purified by silica gel column chromatography (eluent: n-hexane/ethyl acetate=1/1) to obtain 0.035 g (purity: 76.8%, 0.11 mmol) of the title compound as a white solid. The yield was 21.1% (a mixture of isomers; ratio of isomers of the title compound: 97.0:3.0). Incidentally, the compounds contained in impurities were a compound in which the methyl group at the 1-position was epimerized during reaction. The ratio of the title compound and the epimer in the white solid obtained by purification was 82.6:17.4.

¹H NMR (main compound) (500 MHz, CDCl₃) δ:
4.72-4.68 (3H, m), 4.57 (1H, brs), 3.93 (1H, m), 3.69 (1H, m), 2.30-2.14 (4H, m), 2.05 (1H, dd, J=14.3, 9.0 Hz), 1.95-1.90 (2H, m), 1.84-1.75 (2H, m), 1.71 (3H, s), 1.52 (1H, dt, J=12.6, 2.7 Hz), 1.32 (1H, t, J=12.5 Hz), 1.24 (1H, dq, J=12.6, 4.5 Hz), 1.20 (3H, d, J=6.9 Hz)

¹³C NMR (main product) (126 MHz, CDCl₃) δ:
152.93, 149.71, 108.85, 105.07, 83.83, 77.13, 45.29, 43.04, 42.52, 40.57, 40.32, 34.06, 33.18, 21.01, 11.23

GC retention time (measurement condition 3): 11.1 minutes (main product and epimer of methyl group), 11.4 minutes (minor product)

The main product was a compound represented by the following formula (19^{A}):

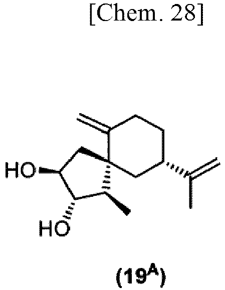

### (Production Example 16)

### Synthesis of (5'S)-2-methyl-2'-methylene-5'-(1-propen-2-yl)-6-oxaspiro[bicyclo[3.1.0]hexane-3,1'-cyclohexane] (compound of the present invention represented by formula (1)) (Eq. 16)

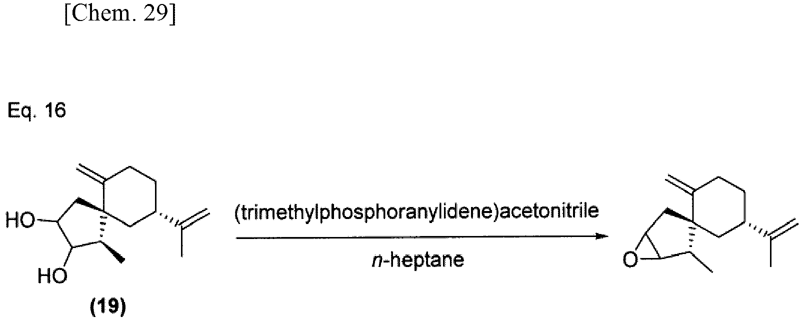

The compound (19) (32.8 mg, purity: 76.8%, 0.11 mmol) obtained in Production Example 15 and dehydrated n-heptane (14 mL) were added to a 30 mL three-necked round-bottom flask, and the mixture was heated using an oil bath. A THF solution (concentration: 0.46 mol/L, 0.9 mL, 0.42 mmol) of (trimethylphospholanylidene)acetonitrile was added dropwise over 5 minutes at an internal temperature of 97 °C, and the solution was stirred at an internal temperature of 94 °C for 30 minutes. The reaction mixture was cooled to room temperature and then concentrated under reduced pressure. The obtained residue was purified by silica gel column chromatography (eluent: n-hexane/ethyl acetate=100/1), as a result, 16.0 mg of a mixture of an isomer (hereinafter, referred to as "compound (1^{A})") of the title compound having a GC retention time of 8.9 minutes and an isomer (hereinafter, referred to as "compound (1^{B})") of the title compound having a GC retention time of 9.0 minutes was obtained as a colorless liquid (purity: 95.9%, 0.070 mmol, ratio of isomers: 84.3 [compound (1^{A})]:15.7 [(compound (1^{B})]), and 4.8 mg of an isomer (hereinafter referred to as "compound (1^{C})") of the title compound having a GC retention time of 9.3 minutes was obtained as a colorless liquid (purity: 91.0%, 0.020 mmol). The yield was 74.3%. Incidentally, the compound having a GC retention time of 9.0 minutes was a compound derived from the epimer of methyl group obtained in Production Example 15.

¹H NMR [compound (1^{A})] (500 MHz, CDCl₃) δ:
4.67 (2H, m), 4.61 (1H, brs), 4.47 (1H, brs), 3.43 (2H, m), 2.53 (1H, d, J=14.4 Hz), 2.51 (1H, q, J=7.3 Hz), 2.28 (1H, m), 2.24 (1H, m), 2.10 (1H, td, J=13.2, 4.0 Hz), 1.86 (1H, dt, J=12.9, 2.8), 1.81 (1H, m), 1.70 (3H, s), 1.48 (1H, dt, J=14.4, 1.3 Hz), 1.20 (1H, m), 1.12 (3H, d, J=7.3 Hz), 1.10 (1H, t, J=13.0 Hz)

¹³C NMR [compound (1^{A})] (126 MHz, CDCl₃) δ:
154.27, 149.82, 108.62, 103.69, 62.62, 55.26, 45.91, 40.96, 40.50, 39.29, 37.54, 34.72, 33.52, 21.15, 10.93

¹H NMR [compound (1^{B})] (500 MHz, CDCl₃) δ:
4.97-4.94 (2H, m), 4.67 (1H, m), 4.66 (1H, brs), 3.43 (1H, m), 3.40 (1H, m), 2.37 (1H, d, J=14.5 Hz), 2.23-2.16 (2H, m), 2.06 (1H, m), 2.01 (1H, dq, J=7.3, 1.3 Hz), 1.77-1.62 (6H, m), 1.37-1.25 (2H, m), 1.19 (3H, d, J=7.4 Hz)

¹³C NMR [compound (1^{B})] (126 MHz, CDCl₃) δ:
150.61, 149.98, 113.36, 108.38, 61.70, 55.52, 47.35, 47.25, 45.90, 42.08, 40.79, 34.10, 30.59, 20.70, 11.92

¹H NMR [compound (1^{C})] (500 MHz, CDCl₃) δ:
4.78 (1H, brs), 4.69 (2H, m), 4.68 (1H, brs), 3.42 (1H, t, J=2.4 Hz), 3.28 (1H, d, J=2.9 Hz), 2.73 (1H, q, J=7.4 Hz), 2.32-2.25 (2H, m), 2.20-2.10 (3H, m), 1.84-1.77 (2H, m), 1.77-1.70 (1H, m), 1.70 (3H, s), 1.57 (1H, m), 1.27-1.15 (3H, m), 0.96 (3H, d, J=7.4 Hz)

¹³C NMR [compound (1^{C})] (126 MHz, CDCl₃) δ:
153.78, 149.94, 108.75, 106.96, 62.18, 56.63, 49.19, 41.95, 41.38, 38.76, 37.18, 34.58, 33.33, 20.74, 12.50

GC retention time (measurement condition 3): 8.9 minutes [compound (1^{A})], 9.0 minutes [compound (1^{B})], 9.3 minutes [compound (1^{C})]

### (Example 2) Grapefruit-Like Flavor composition

A grapefruit-like flavor composition (A) was prepared according to the formulation in Table 1 below.

### [Table 1]

**Table 1: Formulation of Flavor composition (A)**

| Component | parts by mass |
|---|---|
| Compound (1^{C}) obtained in Example 1 | 0.01 |
| Grapefruit oil (cold pressed) | 50 |
| Ethanol | balance |
| Total | 1000 |

### (Comparative Example 1)

As Comparative Example 1, a grapefruit-like flavor composition (B) was prepared according to the formulation in Table 2 below.

### [Table 2]

**Table 2: Formulation of Flavor composition (B)**

| Component | parts by mass |
|---|---|
| Grapefruit oil (cold pressed) | 50 |
| Ethanol | balance |
| Total | 1000 |

### (Test Example 1)

0.1 mass% of each of the grapefruit-like flavor compositions (A) and (B) obtained in Example 2 and Comparative Example 1 was added to water, and sensory evaluation was performed by eight expert panelists. As a result, all panelists answered water which the flavor composition (A) of Example 2 was added thereto was provided with more natural and fresher fruit juice-like feeling than water which the flavor composition (B) of Comparative Example 1 was added thereto, and was far excellent.

### (Example 3) Addition to Commercially Available Grapefruit Juice Beverage

10 ppb of the compound (1^{C}) obtained in Example 1 was added to a commercially available grapefruit juice beverage, and sensory evaluation was performed by eight expert panelists. As a result, all panelists answered the beverage was provided with natural fruit juice-like feeling, rich in voluminous feeling, and was far excellent.

### (Example 4) Grapefruit-Like Flavor composition

A grapefruit-like flavor composition (C) was prepared according to the formulation in Table 3 below.

### [Table 3]

**Table 3: Formulation of Flavor composition (C)**

| Component | parts by mass |
|---|---|
| Compound (1^{C}) obtained in Example 1 | 0.01 |
| Nootkatone | 2 |
| Octanal | 0.3 |
| Decanal | 0.2 |
| Dodecanal | 0.1 |
| Linalool | 0.8 |
| cis-3-Hexenol | 0.5 |
| Ethanol | balance |
| Total | 1000 |

### (Comparative Example 2)

As Comparative Example 2, a grapefruit-like flavor composition (D) was prepared according to the formulation in Table 4 below.

### [Table 4]

**Table 4: Formulation of Flavor composition (D)**

| Component | parts by mass |
|---|---|
| Nootkatone | 2 |
| Octanal | 0.3 |
| Decanal | 0.2 |
| Dodecanal | 0.1 |
| Linalool | 0.8 |
| cis-3-Hexenol | 0.5 |
| Ethanol | balance |
| Total | 1000 |

### (Test Example 2)

0.1 mass% of each of the grapefruit-like flavor compositions (C) and (D) obtained in Example 4 and Comparative Example 2 was added to water, and sensory evaluation was performed by eight expert panelists. As a result, all panelists answered water which the flavor composition (C) of Example 4 was added thereto was provided with natural and rich fruit juice-like feeling unperceivable from water which the flavor composition (D) of Comparative Example 2 was added thereto, and was far excellent.

### (Example 5) Orange-Like Flavor composition

An orange-like flavor composition (E) was prepared according to the formulation in Table 5 below.

### [Table 5]

**Table 5: Formulation of Flavor composition (E)**

| Component | parts by mass |
|---|---|
| Compound (1^{C}) obtained in Example 1 | 0.01 |
| Orange oil (cold pressed) | 50 |
| Ethanol | balance |
| Total | 1000 |

### (Comparative Example 3)

As Comparative Example 3, an orange-like flavor composition (F) was prepared according to the formulation in Table 6 below.

### [Table 6]

**Table 6: Formulation of Flavor composition (F)**

| Component | parts by mass |
|---|---|
| Orange oil (cold pressed) | 50 |
| Ethanol | balance |
| Total | 1000 |

### (Test Example 3)

0.1 mass% of each of the orange-like flavor compositions (E) and (F) obtained in Example 5 and Comparative Example 3 was added to water, and sensory evaluation was performed by eight expert panelists. As a result, all panelists answered water which the flavor composition (E) of Example 5 was added thereto was provided with more natural and fresher fruit juice-like feeling than water which the flavor composition (F) of Comparative Example 3 was added thereto, and was far excellent.

### (Example 6) Addition to Commercially Available Orange Juice Beverage

10 ppb of the compound (1^{C}) obtained in Example 1 was added to a commercially available orange juice beverage, and sensory evaluation was performed by eight expert panelists. As a result, all panelists answered the beverage was provided with natural fruit juice-like feeling, rich in voluminous feeling, and was far excellent.

### (Example 7) Orange-Like Flavor composition

An orange-like flavor composition (G) was prepared according to the formulation in Table 7 below.

### [Table 7]

**Table 7: Formulation of Flavor composition (G)**

| Component | parts by mass |
|---|---|
| Compound (1^{C}) obtained in Example 1 | 0.01 |
| Ethyl butyrate | 0.5 |
| Octanal | 1 |
| Nonanal | 0.1 |
| Decanal | 0.5 |
| Linalool | 2 |
| Ethanol | balance |
| Total | 1000 |

### (Comparative Example 4)

As Comparative Example 4, an orange-like flavor composition (H) was prepared according to the formulation in Table 8 below.

### [Table 8]

**Table 8: Formulation of Flavor composition (H)**

| Component | parts by mass |
|---|---|
| Ethyl butyrate | 0.5 |
| Octanal | 1 |
| Nonanal | 0.1 |
| Decanal | 0.5 |
| Linalool | 2 |
| Ethanol | balance |
| Total | 1000 |

### (Test Example 4)

0.1 mass% of each of the orange-like flavor compositions (G) and (H) obtained in Example 7 and Comparative Example 4 was added to water, and sensory evaluation was performed by eight expert panelists. As a result, all panelists answered water which the flavor composition (G) of Example 7 was added thereto was provided with natural and rich fruit juice-like feeling unperceivable from water which the flavor composition (H) of Comparative Example 4 was added thereto, and was far excellent.

### (Example 8) Lemon-Like Flavor composition

A lemon-like flavor composition (I) was prepared according to the formulation in Table 9 below.

### [Table 9]

**Table 9: Formulation of Flavor composition (I)**

| Component | parts by mass |
|---|---|
| Compound (1^{C}) obtained in Example 1 | 0.01 |
| Lemon oil (cold pressed) | 50 |
| Ethanol | balance |
| Total | 1000 |

### (Comparative Example 5)

As Comparative Example 5, a lemon-like flavor composition (J) was prepared according to the formulation in Table 10 below.

### [Table 10]

**Table 10: Formulation of Flavor composition (J)**

| Component | parts by mass |
|---|---|
| Lemon oil (cold pressed) | 50 |
| Ethanol | balance |
| Total | 1000 |

### (Test Example 5)

0.1 mass% of each of the lemon-like flavor compositions (I) and (J) obtained in Example 8 and Comparative Example 5 was added to water, and sensory evaluation was performed by eight expert panelists. As a result, all panelists answered water which the flavor composition (I) of Example 8 was added thereto was provided with more natural and fresher fruit juice-like feeling than water which the flavor composition (J) of Comparative Example 5 was added thereto, and was far excellent.

### (Example 9) Addition to Commercially Available Lemon Juice Beverage

10 ppb of the compound (1^{C}) obtained in Example 1 was added to a commercially available lemon juice beverage, and sensory evaluation was performed by eight expert panelists. As a result, all panelists answered the beverage was provided with natural fruit juice-like feeling rich in voluminous feeling, and was far excellent.

### (Example 10) Lemon-Like Flavor composition

A lemon-like flavor composition (K) was prepared according to the formulation in Table 11 below.

### [Table 11]

**Table 11: Formulation of Flavor composition (K)**

| Component | parts by mass |
|---|---|
| Compound (1^{C}) obtained in Example 1 | 0.01 |
| Citral | 3 |
| α-Terpineol | 1 |
| Geraniol | 0.5 |
| Geranyl acetate | 0.5 |
| Neryl acetate | 0.5 |
| Ethanol | balance |
| Total | 1000 |

### (Comparative Example 6)

As Comparative Example 6, a lemon-like flavor composition (L) was prepared according to the formulation in Table 12 below.

### [Table 12]

**Table 12: Formulation of Flavor composition (L)**

| Component | parts by mass |
|---|---|
| Citral | 3 |
| α-Terpineol | 1 |
| Geraniol | 0.5 |
| Geranyl acetate | 0.5 |
| Neryl acetate | 0.5 |
| Ethanol | balance |
| Total | 1000 |

### (Test Example 6)

0.1 mass% of each of the lemon-like flavor compositions (K) and (L) obtained in Example 10 and Comparative Example 6 was added to water, and sensory evaluation was performed by eight expert panelists. As a result, all panelists answered water which the flavor composition (K) of Example 10 was added thereto was provided with natural and rich fruit juice-like feeling unperceivable from water which the flavor composition (L) of Comparative Example 6 was added thereto, and was far excellent.

### (Example 11) Addition to Commercially Available Apple Juice Beverage

1 ppb of the compound (1^{C}) obtained in Example 1 was added to a commercially available apple juice beverage, and sensory evaluation was performed by eight expert panelists. As a result, all panelists answered the beverage was provided with natural fruit juice-like feeling accompanied by an impression of firm flesh, and was far excellent.

### (Example 12) Apple-Like Flavor composition

An apple-like flavor composition (M) was prepared according to the formulation in Table 13 below.

### [Table 13]

**Table 13: Formulation of Flavor composition (M)**

| Component | parts by mass |
|---|---|
| Compound (1^{C}) obtained in Example 1 | 0.001 |
| 2-Methylbutyl acetate | 16 |
| Hexanol | 12 |
| trans-2-Hexenal | 1.5 |
| Isoamyl alcohol | 10 |
| Hexanal | 3 |
| Hexyl acetate | 6 |
| Butyl acetate | 12 |
| Ethyl butyrate | 3 |
| Ethyl 2-methylbutyrate | 2 |
| Acetic acid | 4 |
| Damascenone | 0.005 |
| Ethanol | balance |
| Total | 1000 |

### (Comparative Example 7)

As Comparative Example 7, an apple-like flavor composition (N) was prepared according to the formulation in Table 14 below.

### [Table 14]

**Table 14: Formulation of Flavor composition (N)**

| Component | parts by mass |
|---|---|
| 2-Methylbutyl acetate | 16 |
| Hexanol | 12 |
| trans-2-Hexenal | 1.5 |
| Isoamyl alcohol | 10 |
| Hexanal | 3 |
| Hexyl acetate | 6 |
| Butyl acetate | 12 |
| Ethyl butyrate | 3 |
| Ethyl 2-methylbutyrate | 2 |
| Acetic acid | 4 |
| Damascenone | 0.005 |
| Ethanol | balance |
| Total | 1000 |

### (Test Example 7)

0.1 mass% of each of the apple-like flavor compositions (M) and (N) obtained in Example 12 and Comparative Example 7 was added to water, and sensory evaluation was performed by eight expert panelists. As a result, all panelists answered water which the flavor composition (M) of Example 12 was added thereto was provided with more natural flesh-like feeling accompanied by an impression of firm flesh than water which the flavor composition (N) of Comparative Example 7 was added thereto, and was far excellent.

### (Example 13) Addition to Commercially Available Grape Juice Beverage

1 ppb of the compound (1^{C}) obtained in Example 1 was added to a commercially available grape juice beverage, and sensory evaluation was performed by eight expert panelists. As a result, all panelists answered the beverage was provided with rich and natural fruit juice-like feeling and, was far excellent.

### (Example 14)

A grape-like flavor composition (O) was prepared according to the formulation in Table 15 below.

### [Table 15]

**Table 15: Formulation of Flavor composition (O)**

| Component | parts by mass |
|---|---|
| Compound (1^{C}) obtained in Example 1 | 0.001 |
| Ethyl acetate | 30 |
| Ethyl butyrate | 20 |
| cis-3-Hexenol | 8 |
| Methyl anthranilate | 15 |
| Ethyl maltol | 20 |
| Propionic acid | 15 |
| Hexanoic acid | 1.5 |
| trans-2-Hexenal | 2 |
| Propyl acetate | 25 |
| Ethyl propionate | 30 |
| Linalool | 0.3 |
| Cinnamic alcohol | 0.3 |
| Damascenone | 0.002 |
| Ethanol | balance |
| Total | 1000 |

### (Comparative Example 8)

As Comparative Example 8, a grape-like flavor composition (P) was prepared according to the formulation in Table 16 below.

### [Table 16]

**Table 16: Formulation of Flavor composition (P)**

| Component | parts by mass |
|---|---|
| Ethyl acetate | 30 |
| Ethyl butyrate | 20 |
| cis-3-Hexenol | 8 |
| Methyl anthranilate | 15 |
| Ethyl maltol | 20 |
| Propionic acid | 15 |
| Hexanoic acid | 1.5 |
| trans-2-Hexenal | 2 |
| Propyl acetate | 25 |
| Ethyl propionate | 30 |
| Linalool | 0.3 |
| Cinnamic alcohol | 0.3 |
| Damascenone | 0.002 |
| Ethanol | balance |
| Total | 1000 |

### (Test Example 8)

0.1 mass% of each of the grape-like flavor compositions (O) and (P) obtained in Example 14 and Comparative Example 8 was added to water, and sensory evaluation was performed by eight expert panelists. As a result, all panelists answered water which the flavor composition (O) of Example 14 was added thereto was provided with richer and more natural flesh-like feeling than water which the flavor composition (P) of Comparative Example 8 was added thereto, and was far excellent.

### (Example 15) Addition to Commercially Available Peach Juice Beverage

1 ppb of the compound (1^{C}) obtained in Example 1 was added to a commercially available peach juice beverage, and sensory evaluation was performed by eight expert panelists. As a result, all panelists answered the beverage was provided with natural flesh-like feeling accompanied by an image of fiber, and was far excellent.

### (Example 16)

A peach-like flavor composition (Q) was prepared according to the formulation in Table 17 below.

### [Table 17]

**Table 17: Formulation of Flavor composition (Q)**

| Component | parts by mass |
|---|---|
| A mixture of compound (1^{A}) and compound (1^{B}) obtained in Example 1 (1^{A}: 1^{B}=84.3: 15.7) | 0.001 |
| Ethyl acetate | 50 |
| Hexanol | 1 |
| Benzaldehyde | 0.4 |
| Maltol | 2 |
| Hexanal | 0.2 |
| Hexyl acetate | 3 |
| γ-Undecalactone | 0.3 |
| Ethyl octanoate | 0.05 |
| β-Ionone | 0.002 |
| Linalool | 0.2 |
| Isoamyl acetate | 8 |
| Ethyl butyrate | 3 |
| Ethanol | balance |
| Total | 1000 |

### (Comparative Example 9)

As Comparative Example 9, a peach-like flavor composition (R) was prepared according to the formulation in Table 18 below.

### [Table 18]

**Table 18: Formulation of Flavor composition (R)**

| Component | parts by mass |
|---|---|
| Ethyl acetate | 50 |
| Hexanol | 1 |
| Benzaldehyde | 0.4 |
| Maltol | 2 |
| Hexanal | 0.2 |
| Hexyl acetate | 3 |
| γ-Undecalactone | 0.3 |
| Ethyl octanoate | 0.05 |
| β-Ionone | 0.002 |
| Linalool | 0.2 |
| Isoamyl acetate | 8 |
| Ethyl butyrate | 3 |
| Ethanol | balance |
| Total | 1000 |

### (Test Example 9)

0.1 mass% of each of the peach-like flavor compositions (Q) and (R) obtained in Example 16 and Comparative Example 9 was added to water, and sensory evaluation was performed by eight expert panelists. As a result, all panelists answered water which the flavor composition (Q) of Example 16 was added thereto was provided with more natural flesh-like feeling accompanied by an image of fiber than water which the flavor composition (R) of Comparative Example 9 was added thereto, and was far excellent.

### (Example 17) Addition to Commercially Available Wine Taste Beverage

1 ppb of a mixture of compound (1^{A}) and compound (1^{B}) obtained in Example 1 was added to a commercially available wine taste beverage, and sensory evaluation was performed by eight expert panelists. As a result, all panelists answered the beverage was enhanced in natural oaky note and ripened sensation, and was far excellent.

### (Example 18)

A wine-like flavor composition (S) was prepared according to the formulation in Table 19 below.

### [Table 19]

**Table 19: Formulation of Flavor composition (S)**

| Component | parts by mass |
|---|---|
| A mixture of compound (1^{A}), compound (1^{B}) and compound (1^{C}) obtained in Example 1; mass ratio (1^{A}: 1^{B}: 1^{C}=75.9: 14.1: 10.0) | 0.001 |
| Isoamyl acetate | 3 |
| Ethyl hexanoate | 1 |
| Ethyl octanoate | 2 |
| Ethyl decanoate | 0.6 |
| 2-Phenylethyl alcohol | 1 |
| Octanoic acid | 1 |
| Decanoic acid | 0.8 |
| Damascenone | 0.005 |
| Ethyl 2-methylbutyrate | 0.05 |
| Ethyl butyrate | 0.3 |
| Geraniol | 0.02 |
| Linalool | 0.03 |
| 2-Phenylethyl acetate | 0.5 |
| Ethyl acetate | 35 |
| Isoamyl alcohol | 40 |
| Dimethyl sulfide | 0.03 |
| Ethanol | balance |
| Total | 1000 |

### (Comparative Example 10)

As Comparative Example 10, a wine-like flavor composition (T) was prepared according to the formulation in Table 20 below.

### [Table 20]

**Table 20: Formulation of Flavor composition (T)**

| Component | parts by mass |
|---|---|
| Isoamyl acetate | 3 |
| Ethyl hexanoate | 1 |
| Ethyl octanoate | 2 |
| Ethyl decanoate | 0.6 |
| 2-Phenylethyl alcohol | 1 |
| Octanoic acid | 1 |
| Decanoic acid | 0.8 |
| Damascenone | 0.005 |
| Ethyl 2-methylbutyrate | 0.05 |
| Ethyl butyrate | 0.3 |
| Geraniol | 0.02 |
| Linalool | 0.03 |
| 2-Phenylethyl acetate | 0.5 |
| Ethyl acetate | 35 |
| Isoamyl alcohol | 40 |
| Dimethyl sulfide | 0.03 |
| Ethanol | balance |
| Total | 1000 |

### (Test Example 10)

0.1 mass% of each of the wine-like flavor compositions (S) and (T) obtained in Example 18 and Comparative Example 10 was added to water, and sensory evaluation was performed by eight expert panelists. As a result, all panelists answered water which the flavor composition (S) of Example 18 was more enhanced in the natural oaky note and ripened sensation than water which the flavor composition (T) of Comparative Example 10 was added thereto, and was far excellent.

### (Test Example 11)

0.1 mass% of each of the grapefruit-like flavor compositions (A) and (B) obtained in Example 2 and Comparative Example 1 was added to a commercially available grapefruit juice beverage, and sensory evaluation was performed by eight expert panelists. As a result, all panelists answered the grapefruit juice beverage which the flavor composition (A) of Example 2 was added thereto was provided with more natural fruit juice-like feeling and rich in voluminous feeling than the grapefruit juice beverage which the flavor composition (B) of Comparative Example 1 was added thereto, and was far excellent.

### (Test Example 12)

0.1 mass% of each of the grapefruit-like flavor compositions (C) and (D) obtained in Example 4 and Comparative Example 2 was added to a commercially available grapefruit juice beverage, and sensory evaluation was performed by eight expert panelists. As a result, all panelists answered the grapefruit juice beverage which the flavor composition (C) of Example 4 was added thereto was provided with more natural fruit juice-like feeling and rich in voluminous feeling than the grapefruit juice beverage which the flavor composition (D) of Comparative Example 2 was added thereto, and was far excellent.

### (Test Example 13)

0.1 mass% of each of the orange-like flavor compositions (E) and (F) obtained in Example 5 and Comparative Example 3 was added to a commercially available orange juice beverage, and sensory evaluation was performed by eight expert panelists. As a result, all panelists answered the orange juice beverage which the flavor composition (E) of Example 5 was added thereto was provided with more natural fruit juice-like feeling and rich in voluminous feeling than the orange juice beverage which the flavor composition (F) of Comparative Example 3 was added thereto, and was far excellent.

### (Test Example 14)

0.1 mass% of each of the orange-like flavor compositions (G) and (H) obtained in Example 7 and Comparative Example 4 was added to a commercially available orange juice beverage, and sensory evaluation was performed by eight expert panelists. As a result, all panelists answered the orange juice beverage which the flavor composition (G) of Example 7 was added thereto was provided with more natural fruit juice-like feeling and rich in voluminous feeling than the orange juice beverage which the flavor composition (H) of Comparative Example 4 was added thereto, and was far excellent.

### (Test Example 15)

0.1 mass% of each of the lemon-like flavor compositions (I) and (J) obtained in Example 8 and Comparative Example 5 was added to a commercially available lemon juice beverage, and sensory evaluation was performed by eight expert panelists. As a result, all panelists answered the lemon juice beverage which the flavor composition (I) of Example 8 was added thereto was provided with more natural fruit juice-like feeling and rich in voluminous feeling than the lemon juice beverage which the flavor composition (J) of Comparative Example 5 was added thereto, and was far excellent.

### (Test Example 16)

0.1 mass% of each of the lemon-like flavor compositions (K) and (L) obtained in Example 10 and Comparative Example 6 was added to a commercially available lemon juice beverage, and sensory evaluation was performed by eight expert panelists. As a result, all panelists answered the lemon juice beverage which the flavor composition (K) of Example 10 was added thereto was provided with more natural fruit juice-like feeling and rich in voluminous feeling than the lemon juice beverage which the flavor composition (L) of Comparative Example 6 was added thereto, and was far excellent.

### (Test Example 17)

0.1 mass% of each of the apple-like flavor compositions (M) and (N) obtained in Example 12 and Comparative Example 7 was added to a commercially available apple juice beverage, and sensory evaluation was performed by eight expert panelists. As a result, all panelists answered the apple juice beverage which the flavor composition (M) of Example 12 was added thereto was provided with more natural flesh-like feeling accompanied by an impression of firm flesh than the apple juice beverage which the flavor composition (N) of Comparative Example 7 was added thereto, and was far excellent.

### (Test Example 18)

0.1 mass% of each of the grape-like flavor compositions (O) and (P) obtained in Example 14 and Comparative Example 8 was added to a commercially available grape juice beverage, and sensory evaluation was performed by eight expert panelists. As a result, all panelists answered the grape juice beverage which the flavor composition (O) of Example 14 was added thereto was provided with richer and more natural fruit juice-like feeling than the grape juice beverage which the flavor composition (P) of Comparative Example 8 was added thereto, and was far excellent.

### (Test Example 19)

0.1 mass% of each of the peach-like flavor compositions (Q) and (R) obtained in Example 16 and Comparative Example 9 was added to a commercially available peach juice beverage, and sensory evaluation was performed by eight expert panelists. As a result, all panelists answered the peach juice beverage which the flavor composition (Q) of Example 16 was added thereto was provided with more natural flesh-like feeling accompanied by an image of fiber than the peach juice beverage which the flavor composition (R) of Comparative Example 9 was added thereto, and was far excellent.

### (Test Example 20)

0.1 mass% of each of the wine-like flavor compositions (S) and (T) obtained in Example 18 and Comparative Example 10 was added to a commercially available wine taste juice beverage, and sensory evaluation was performed by eight expert panelists. As a result, all panelists answered the wine taste juice beverage which the flavor composition (S) of Example 18 was added thereto was more enhanced in natural oaky note and ripened sensation than the wine taste juice beverage which the flavor composition (T) of Comparative Example 10 was added thereto, and was far excellent.

While the invention has been described in detail and with reference to specific embodiments thereof, it will be apparent to one skilled in the art that various changes and modifications can be made therein without departing from the spirit and scope of the invention. This application is based on Japanese Patent Application (Patent Application No. 2017-034869) filed on February 27, 2017, the contents of which are incorporated herein by way of reference.

### INDUSTRIAL APPLICABILITY

The compound represented by formula (1) of the present invention is superior for imparting or enhancing the aroma and flavor giving natural fruity feeling, fruit juice-like feeling or ripened sensation. Therefore, the compound represented by formula (1) of the present invention can be effectively used for a food/beverage directly by itself or in the form of a flavor composition and can impart or enhance the aroma and flavor giving natural fruity feeling, fruit juice-like feeling or ripened sensation to the food/beverage.

## Claims

1. A compound represented by the following formula (1):

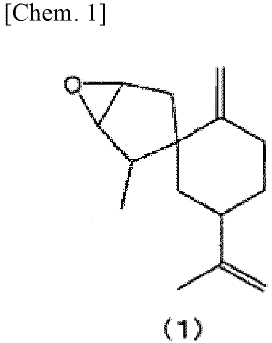

2. A flavor composition containing the compound according to claim 1.

3. The flavor composition according to claim 2, which is a flavor composition having a fruit-like aroma.

4. The flavor composition according to claim 3, wherein the fruit-like aroma is a citrus fruit aroma.

5. The flavor composition according to claim 4, wherein the citrus fruit is at least one selected from the group consisting of grapefruit, orange and lemon.

6. The flavor composition according to any one of claims 2 to 5, which is a flavor composition for a food/beverage.

7. The flavor composition according to claim 6, wherein the food/beverage is a beverage.

8. The flavor composition according to claim 7, wherein the beverage is a citrus beverage.

9. A food/beverage containing the compound according to claim 1.

10. A food/beverage containing the flavor composition according to any one of claims 2 to 8.

11. A method for producing a food/beverage, comprising adding the compound according to claim 1.

12. A method for producing a food/beverage, comprising adding the flavor composition according to any one of claims 2 to 8.
